(19) Europäisches Patentamt
European Patent Office
Office européen des brevets

(11) EP 2 793 568 B1

# (12) EUROPEAN PATENT SPECIFICATION

(45) Date of publication and mention of the grant of the patent:
**13.06.2018 Bulletin 2018/24**

(21) Application number: **12799082.8**

(22) Date of filing: **12.10.2012**

(51) Int Cl.:
***A01K 67/033*** *(2006.01)* ***A61K 31/7088*** *(2006.01)*
***A61K 39/00*** *(2006.01)* ***A61K 39/395*** *(2006.01)*
***C07K 14/00*** *(2006.01)* ***C07K 14/435*** *(2006.01)*
***C12P 21/00*** *(2006.01)* ***C12Q 1/68*** *(2018.01)*
***G01N 33/567*** *(2006.01)*

(86) International application number:
**PCT/EP2012/070246**

(87) International publication number:
**WO 2013/091924 (27.06.2013 Gazette 2013/26)**

## (54) APPLICATIONS OF AN IMMUNE SYSTEM-RELEASED ACTIVATING AGENT (ISRAA)

ANWENDUNGEN EINES IM IMMUNSYSTEM-FREIGESETZTEN AKTIVIERUNGSMITTELS

APPLICATIONS D'UN AGENT D'ACTIVATION LIBÉRÉ PAR LE SYSTÈME IMMUNITAIRE (ISRAA)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2011 GB 201121891**

(43) Date of publication of application:
**29.10.2014 Bulletin 2014/44**

(73) Proprietors:
- **Arabian Gulf University**
  **Manama (BH)**
- **Bakhiet, Abdelmoiz**
  **Manama (BH)**

(72) Inventors:
- **BAKHIET, Abdelmoiz**
  **University Manama**
  **P.O.Box 26671 (BH)**
- **TAHA,Safa**
  **University Manama**
  **P.O Box 26671 (BH)**

(74) Representative: **Gill, Siân Victoria et al**
**Venner Shipley LLP**
**200 Aldersgate**
**London EC1A 4HD (GB)**

(56) References cited:
**WO-A2-2008/104575 US-A1- 2007 237 770**

- **MOIZ BAKHIET ET AL: "A novel nervous system-induced factor inducing immune responses in the spleen", IMMUNOLOGY AND CELL BIOLOGY, vol. 86, no. 8, 9 September 2008 (2008-09-09), pages 688-699, XP55052862, ISSN: 0818-9641, DOI: 10.1038/icb.2008.57 & DATABASE EMBL [Online] 8 April 2008 (2008-04-08), "Mus musculus immune system released activating agent (Israa) mRNA, complete cds.", retrieved from EBI accession no. EMBL:EU552928 Database accession no. EU552928**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 793 568 B1

## Description

### Technical field

The present invention relates to applications of an immune system-released activating agent (ISRAA) polypeptide, which is induced by a nervous stimulus and which has been found to mediate the transmission of signals between the immune system and the nervous system following an immune challenge. In particular aspects, the present invention concerns the therapeutic applications associated with the activity of the ISRAA polypeptide and related regulatory agents.

### Background Art

A bewildering array of infectious agents and parasites gain subsistence at the expense of their hosts. Although host-parasite interplay depends on the virulence of parasites and resistance of the host, the early events of innate immunity during host-parasite interactions are very important in directing the ultimate pattern of the immune response. These early events of the innate immune response are, however, much less well characterized than the later secondary immune responses.

Immune responses are not isolated from other organ systems in the body, and indeed there are various means of communication between these organ systems and the immune system. Among these communications are regulatory interactions between the nervous system and the immune system. The nervous system of vertebrates is divided into the central nervous system (CNS), consisting of the brain and spinal cord, and the peripheral nervous system (PNS), consisting of all the other nerves that do not form part of the CNS. The PNS is further sub-divided into sympathetic, parasympathetic and enteric systems. The sympathetic nervous system prepares the body for stressful situations and activates what is often termed the 'fight or flight' response. This response is also known as the sympatho-adrenal response of the body, as adrenergic nerves release noradrenaline and adrenaline as neurotransmitters for the sympathetic nervous system. Channels of contact between the nervous system and the immune system include noradrenergic sympathetic innervation of the primary and secondary lymphoid organs in addition to stimulation of lymphocytes and other immuno-competent cells by virtue of their $\beta$- and $\alpha$-adrenergic receptors.

The nervous-to-immune signalling pathway of early natural responses to infection has previously been investigated and the spleen has been shown to play a major role. The spleen, which is an important lymphoid organ involved in immune responses against all types of antigen that appear in the circulation, is richly innervated by adrenergic neurons that enter its parenchyma and remain largely associated with the splenic vasculature.

The induction of chemokines in splenocytes following infection with *Trypanosoma brucei brucei* was examined in Liu, Y. et al (1999a), Tropical Medicine and International Health, 4(2): 85-92. Infection with *Trypanosoma brucei brucei* caused upregulation of particular chemokines, suggesting that these chemokines play a role in early immunopathology. However, splenic denervation of the spleen was shown to suppress this cytokine expression, suggesting that the sympathetic nervous system may participate in the modulation of early immune responses.

Furthermore, Liu, Y. et al (1999b), Scand. J. Immunol. 50: 485-491 demonstrated a regulatory role for the autonomic nervous system on cytokine responses at both mRNA levels and protein levels, as sympathetic denervation of the spleen markedly reduced the mRNA and protein levels of IFN-$\gamma$ and IL-12.

Liu, Y. et al (2004), Neuroimmunomodulation, 11(2): 113-118 studied the effects of splenic sympathectomy on mRNA gene expression and protein production of IL-1$\beta$ and IL-6 in splenic and peritoneal macrophages of rats infected with *Trypanosoma brucei brucei* and non-infected rats. The enhancements of mRNA gene expression and production of IL-1$\beta$ and IL-6 by peritoneal macrophages were significantly suppressed by the splenic denervation in both infected and non-infected rats, indicating a stimulatory role for the sympathetic nervous system during the host immune response in both normal and infected rats.

Critical interactions between the nervous system and the immune system were further investigated in Bakhiet, M. et al, (2006) Clin. Exp. Immunol. 144(2): 290-298 during experimental autoimmune Myasthenia gravis (EAMG). Substantial effects of the nervous system on immune responses that influence the outcome of EAMG were shown, as surgical denervation of the spleen significantly reduced the clinical severity of the disease, suppressed the numbers of IgG and IFN-$\gamma$-secreting cells, downregulated the mRNA expression for cytokines and reduced corticosterone and PGE2 production.

This research has shown that signals generated as a result of contacts between parasites or challenging factors and nerve endings at innate barriers, such as the skin and mucous membranes, are transmitted by sympathetic or parasympathetic nerves to the spleen. It has, therefore, been suggested that the spleen accommodates a central control unit (CCU) for the immune system that receives signals during dangerous challenges, such as parasitic infections, and assumes a coordination function in mobilising defense mechanisms. Without being limited to any particular theory, one current hypothesis is that when the CCU of the spleen receives these alarm signals it gives orders in the form of released mediators to launch primary responses by cells involved in innate immunity in response to these mediators after the host is challenged with infections.

The ISRAA molecule has been recently identified by Bakhiet et al (Immunol Cell Biol (2008), 86(8): p. 688-99) as a 15 kDa novel nervous system-induced factor, inducing immune responses in the spleen. ISRAA gene was identified and further cloned and its sequence was mapped to chromosome 14 in the mouse system.

WO2008/10475 discloses an isolated ISRAA polypeptide having a mediation role in transmission of signals between the nervous system and the immune system following an immune challenge.

**Statement of invention**

The present invention is based upon effects that have heretofore not been observed with the ISRAA molecule, as defined herein and which offers significant potential as a therapeutic agent.

In accordance with a first aspect of the present invention, there is provided an isolated polypeptide molecule, having the amino acid sequence of SEQ ID NO: 3, for use in the treatment of: a) cancer; b) neurological diseases and disorders; or c) muscular diseases and disorders; wherein (i) the effective dose for treatment of cancer is at a concentration of 50 $\mu$g/ml; and (ii) the effective dose for treatment of neurological diseases and disorders or muscular diseases and disorders is at a concentration of 500 pg/ml.

The present invention also concerns potential therapeutic effects based upon the stimulating effect of the ISRAA polypeptide on differentiation of peripheral blood stem cells to nerve and muscle cells or for differentiation of embryonic brain cells to nerve and glial cells.

The present invention further concerns therapeutic applications, pharmaceutical compositions and kits associated with apoptotic activity exhibited by the ISRAA polypeptide on tumour cells.

Titration of ISRAA activity on human cells showed a dose-dependent dualistic effect: an apoptotic effect on tumour cells and proliferative effects on normal immune cells, e.g. Peripheral Blood Mononuclear Cells (PBMC). Thus, the present invention provides for compounds and pharmaceutical compositions for treatment of cancer through an apoptotic effects on tumour cells.

In particular aspects, the present disclosure concerns the therapeutic applications associated with the activity of the ISRAA polypeptide and related regulatory and inhibitory agents such as for use in a method of treating of cancer, immunosuppression, immunodeficiency, an inflammatory and autoimmune diseases, pathogenic infections, neurologic or muscular diseases and disorders. More specifically such therapeutic applications concern use for method of (i) treating of immunosuppression due to HIV/ AIDS, cancer, leukemia, bone marrow depression due to aplastic anaemia or diabetes; (ii) for treating neuromuscular disorders such as Alzheimers Disease , Parkinson's disease, Amyotrophic Lateral Sclerosis (ALS),

Muscular dystrophies; or diabetes; or (iii) for use in treatment of inflammatory and auto immune diseases such as Multiple sclerosis, Myasthenia gravis , Guillain-Barre syndrome; Hashimoto's thyroiditis, Rheumatoid arthritis and Systemic lupus erythematosus (SLE) and type 1 diabetes.

**Specific Description of the Invention**

In accordance with a first aspect of the present invention, there is provided an isolated polypeptide, designated herein as ISRAA (Immune System-Released Activating agent) for use in a method of treatment of patients with cancer, neurological diseases and disorders; or muscular diseases and disorders. The ISRAA polypeptide has advantageously been found to play an important mediation role in transmission of signals between the nervous system and the immune system. The polypeptide for use in accordance with the present invention has the amino acid sequence according to SEQ ID No: 3 and may be encoded by a nucleic acid molecule with the full length nucleotide sequence of *israa* gene (SEQ ID No: 1) or comprising the nucleotide sequence of the open-reading frame identified in SEQ ID No: 2.

The term "isolated" refers to a polypeptide substantially free of cellular material or culture medium when produced by recombinant DNA techniques, or chemical precursors or other chemicals when chemically synthesized. The term "nucleic acid" molecule is intended to include DNA and RNA and can be either double stranded or single stranded.

Homology refers to sequence similarity between sequences and can be determined by comparing a position in each sequence which may be aligned for purposes of comparison. When a position in the compared sequence is occupied by the same nucleotide base or amino acid, then the molecules are homologous at that position. A degree of homology between sequences is a function of the number of matching or homologous positions shared by the sequences.

The term "sequences having substantial sequence homology" means those nucleotide and amino acid sequences which have slight or inconsequential sequence variations from the sequences disclosed in SEQ ID No:1 and SEQ ID No: 2, i.e. the homologous nucleic acids function in substantially the same manner to produce substantially the same polypeptides as the actual sequences. Alternative splice variants corresponding to a cDNA of the invention are also encompassed.

"Percent (%) amino acid sequence identity" with respect to the ISRAA polypeptide sequences identified herein is defined as the percentage of amino acid residues in a candidate sequence that are identical with the amino acid residues in the specific ISRAA polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared.

Percent amino acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span=1, overlap fraction=0.125, word threshold (T)=11, and scoring matrix=BLOSUM62. When WU-BLAST-2 is employed, a % amino acid sequence identity value is determined by dividing (a) the number of matching identical amino acid residues between the amino acid sequence of the ISRAA polypeptide of interest having a sequence derived from the native ISRAA polypeptide and the comparison amino acid sequence of interest (i.e., the sequence against which the ISRAA polypeptide of interest is being compared which may be a ISRAA variant polypeptide) as determined by WU-BLAST-2 by (b) the total number of amino acid residues of the ISRAA polypeptide of interest. For example, in the statement "a polypeptide comprising an the amino acid sequence A which has or having at least 80% amino acid sequence identity to the amino acid sequence B", the amino acid sequence A is the comparison amino acid sequence of interest and the amino acid sequence B is the amino acid sequence of the ISRAA polypeptide of interest.

Percent amino acid sequence identity may also be determined using the sequence comparison program NCBI-BLAST2 (Altschul et al, Nucleic Acids Res. 25:3389-3402 (1997)). NCBI-BLAST2 uses several search parameters, wherein all of those search parameters are set to default values including, for example, unmask=yes, strand=all, expected occurrences=10, minimum low complexity length=15/5, multipass e-value=0.01, constant for multi-pass=25, dropoff for final gapped alignment=25 and scoring matrix=BLOSUM62

Isolated nucleic acids encoding the polypeptide for use according to the present invention, and having a sequence which differs from a nucleotide sequence shown in SEQ ID NO: 1 or 2 due to degeneracy in the genetic code are also disclosed. Such nucleic acids encode functionally equivalent proteins (e.g., a protein induced by the first nervous-to-immune signal) but differ in sequence from the sequence of SEQ ID NO: 1 or 2 due to degeneracy in the genetic code. Degeneracy means that a number of amino acids are designated by more than one triplet. DNA sequence polymorphisms that do lead to changes in the amino acid sequences of an ISRAA protein will also exist within a population. Any and all such nucleotide variations and resulting amino acid polymorphisms are disclosed.

As previously described, recombinant vectors comprising nucleic acid molecules that encode the ISRAA polypeptide can be used to produce the polypeptide for use in accordance with the present invention. These recombinant vectors may be plasmids. These recombinant vectors are prokaryotic or eukaryotic expression vectors. The nucleic acid coding for the ISRAA polypeptide may also be operatively linked to a regulatory control sequence.

The nucleic acids which encode the ISRAA polypeptides can be incorporated into a recombinant expression vector using techniques known in the art, thus ensuring good expression of the encoded protein or part thereof. The recombinant expression vectors are "suitable for transformation of a host cell", which means that the recombinant expression vectors contain a nucleic acid or an oligonucleotide fragment thereof of the invention in addition to a regulatory sequence, selected on the basis of the host cells to be used for expression, which is operatively linked to the nucleic acid or oligonucleotide fragment. Operatively linked is intended to mean that the nucleic acid is linked to a regulatory sequence in a manner which allows expression of the nucleic acid. Therefore, nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice. Regulatory sequences are art-recognized and are selected to direct expression of the desired protein in an appropriate host cell. Accordingly, the term regulatory sequence includes promoters, enhancers and other expression control elements. Such regulatory sequences are known to those skilled in the art.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Expression of these recombinant expression vectors is carried out in prokaryotic or eukaryotic cells using standard molecular biology techniques.

The recombinant expression vectors can be used to make a transformant host cell including the recombinant expression vector. The term "transformant host cell" is intended to include prokaryotic and eukaryotic cells which have been transformed or transfected with a recombinant expression vector of the invention. The terms "transformed with", "transfected with", "transformation" and "transfection" are intended to encompass introduction of nucleic acid (e.g. a vector) into a cell by one of many possible techniques known in the art. Prokaryotic cells can be transformed with nucleic acid by, for example, electroporation or calcium-chloride mediated transformation. Nucleic acid can be introduced into mammalian cells via conventional techniques such as calcium phosphate or calcium chloride co-precipitation, DEAE-dextran-mediated transfection, lipofectin, electroporation, microinjection or any other known technique.

A nucleic acid molecule is a "polynucleotide" which is a single- or double-stranded polymer of deoxyribonucleotide or ribonucleotide bases read from the 5' to the 3' end. Polynucleotides include RNA and DNA, and may be isolated from natural sources, synthesized in vitro, or prepared from a combination of natural and synthetic molecules. Sizes of polynucleotides are expressed as base pairs (abbreviated "bp"), nucleotides ("nt"), or kilobases ("kb"). Where the context allows, the latter two terms may describe polynucleotides that are single-stranded or double-stranded. When the term is applied to double-stranded molecules it is used to denote overall length and will be understood to be equivalent to the term "base pairs". It will be recognized by those skilled in the art that the two strands of a double-stranded polynucleotide may differ slightly in length and that the ends thereof may be staggered as a result of enzymatic cleavage; thus all nucleotides within a double-stranded polynucleotide molecule may not be paired.

"Probes and/or primers" as used herein can be RNA or DNA. DNA can be either cDNA or genomic DNA. Polynucleotide probes and primers are single or double-stranded DNA or RNA, generally synthetic oligonucleotides, but may be generated from cloned cDNA or genomic sequences or its complements. Analytical probes will generally be at least 20 nucleotides in length, although somewhat shorter probes (14-17 nucleotides) can be used. PCR primers are at least 5 nucleotides in length, preferably 15 or more nt, more preferably 20-30 nt. Short polynucleotides can be used when a small region of the gene is targeted for analysis. For gross analysis of genes, a polynucleotide probe may comprise an entire exon or more. Probes can be labeled to provide a detectable signal, such as with an enzyme, biotin, a radionuclide, fluorophore, chemiluminescer, paramagnetic particle and the like, which are commercially available from many sources, such as Molecular Probes, Inc., Eugene, OR, and Amersham Corp., Arlington Heights, IL, using techniques that are well known in the art.

In a preferred embodiment, the isolated polypeptide for use in accordance with the present invention, designated herein as ISRAA, with the amino acid sequence according to SEQ ID No: 3 is encoded by a nucleic acid molecule characterised with at least any of 75%, 80%, 85%, 90%, 95% or 100% sequence identity to the sequence represented by the nucleotide sequence of SEQ ID No: 1 or 2.

A "polypeptide" is a polymer of amino acid residues joined by peptide bonds, whether produced naturally or synthetically. Polypeptides of less than about 10 amino acid residues are commonly referred to as "peptides". A "protein" is a macromolecule comprising one or more polypeptide chains. A protein may also comprise non-peptidic components, such as carbohydrate groups. Carbohydrates and other non-peptidic substituents may be added to a protein by the cell in which the protein is produced, and will vary with the type of cell. Proteins are defined herein in terms of their amino acid backbone structures; substituents such as carbohydrate groups are generally not specified, but may be present nonetheless.

A limited number of non-conservative amino acids, amino acids that are not encoded by the genetic code, non-naturally occurring amino acids, and unnatural amino acids may be substituted for zalphal amino acid residues.

Essential amino acids in the polypeptides for use in accordance with the present invention can be identified according to procedures known in the art, such as site-directed mutagenesis or alanine-scanning mutagenesis (Cunningham and Wells, Science 244: 1081-5, 1989; Bass et al., Proc. Natl. Acad. Sci. USA 88:4498-502, 1991). In the latter technique, single alanine mutations are introduced at every residue in the molecule, and the resultant mutant molecules are tested for biological activity (e.g. ligand binding and signal transduction) as disclosed below to identify amino acid residues that are critical to the activity of the molecule. See also, Hilton et al., J. Biol. Chem. 271:4699-4708, 1996 . Sites of ligand-receptor, protein-protein or other biological interaction can also be determined by physical analysis of structure, as determined by such techniques as nuclear magnetic resonance, crystallography, electron diffraction or photoaffinity labeling, in conjunction with mutation of putative contact site amino acids. See, for example, de Vos et al., Science 255:306-312, 1992 ; Smith et al., J. Mol. Biol. 224:899-904, 1992 ; Wlodaver et al., FEBS Lett. 309:59-64, 1992 . The identities of essential amino acids can also be inferred from analysis of homologies with related receptors.

Determination of amino acid residues that are within regions or domains that are critical to maintaining structural integrity can be determined. Within these regions one can determine specific residues that will be more or less tolerant of change and maintain the overall tertiary structure of the molecule. Methods for analyzing sequence structure include, but are not limited to, alignment of multiple sequences with high amino acid or nucleotide identity and computer analysis using available software (e.g., the Insight II viewer and homology modeling tools; MSI, San Diego, CA), secondary structure propensities, binary patterns, complementary packing and buried polar interactions (Barton, Current Opin. Struct. Biol. 5:372-376, 1995; and, Cordes et al., Current Opin. Struct. Biol. 6: 3-10, 1996). In general, when designing modifications to molecules or identifying specific fragments determination of structure will be accompanied by evaluating activity of modified molecules.

The isolated recombinant ISRAA polypeptide (rISRAA) for use in accordance with the present invention may be prepared by culturing a transformed host cell, including a recombinant expression vector, in a suitable medium until said polypeptide is formed, and subsequently isolating the polypeptide. The steps in such a method represent standard laboratory techniques.

The nucleic acid or oligonucleotide molecule may be introduced into the host cell using at least one delivery vehicle or technique known in the art. These techniques include viral vectors, calcium phosphate co-precipitation, microinjection, electroporation, liposome-mediated gene transfer and aerosol delivery.

In one embodiment of the invention there is provided use of the ISRAA polypeptide for treatment of cancer, which is achieved by an apoptotic activity of the polypeptide on cancer cells.

In a further aspect, the invention provides a method for inhibiting the proliferation of cancer cells, comprising contacting with or introducing into said cells the polypeptide or pharmaceutical composition for use according to the present invention.

In another embodiment of the invention, there is provided a use of the ISRAA polypeptide for treatment of neurologic diseases and disorders, which is achieved by stimulation of the differentiation of peripheral blood stem cells or embryonic brain cells to nerve and glial cells.

In yet another embodiment of the invention, there is provided a use of the ISRAA polypeptide for treatment of muscular diseases and disorders, which is achieved by stimulation of the differentiation of peripheral blood stem cells to muscle cells.

In a further aspect, the invention provides a method for stimulating *in vitro* the differentiation of the peripheral blood stem cells into nerve and muscle cells, comprising contacting with or introducing into said cells the polypeptide or pharmaceutical composition for use according to the present invention.

In yet another aspect, the invention provides a method for stimulating *in vitro* the differentiation of embryonic brain cells into nerve and glial cells, comprising contacting with or introducing into said cells the polypeptide or pharmaceutical composition for use according to the present invention.

In another aspect of the invention, the invention relies on the newly discovered dualistic activity of the ISRAA polypeptide, characterised by (i) an apoptotic activity on tumour cells, and (ii) a proliferative activity on normal immune cells and (iii) differentiation activity on peripheral blood stem cells and embryonic brain cells. Titration of the ISRAA activity on human cells showed that the dualistic effect is dose dependent, wherein the apoptotic effect of the polypeptide is achieved at higher concentration than the effective concentration of the polypeptide producing a proliferative effect. In one preferred embodiment of the invention the effective dose for achieving an apoptotic effect is at 50μg/ml. In another embodiment of the invention the effective dose for achieving a proliferative effects, at 500pg/ml.

The different effects of the ISRAA molecule have been achieved at different concentrations, i.e. at 5000 ng (5 μg) per $1x10^5$ cells in 100 μl (high concentration) the molecule induced apoptosis and the 50 pg per $1x10^5$ cells in 100 μl (low concentration) triggered cell activation. Without being bound by theory, this may be explained by the fact that the cell may have several different receptors that bind the same molecule with different affinities and activate different signal transduction pathways to produce different effects. The high concentration of ISRAA may therefore provide the molecule with a chance of binding to different receptors with a high affinity to death domains since this was the major effect with such concentration. Dilution of ISRAA to the low concentration probably resulted in dissociation of the binding to those death domains and generated optimal conditions for binding to other receptors that induced intracellular signalling pathways for cell activation.

Thus, the ISRAA protein can advantageously generate the opposite effects on cells and participates in the first step of a process that can signal a diverse range of activities, including cellular proliferation, or death by apoptosis.

ISRAA protein can be considered as a regulatory protein, which is characterized by the set of its binding domains. These domains are used to construct clusters of different compositions and properties; cellular response depends on the characteristics of the population of possible clusters. Molecular cluster formation is the driving force behind all processes in the cell.

Upon stimulation, the cell receptors are able to form clusters with the same group of signalling proteins. However, the formation of clusters of these receptors can promote two different cell fates: proliferation or death. There also exist situations in which the same stimulus, mediated by the same member of the TNF receptor family, can trigger either proliferation or apoptosis. One of the reasons for this apparently aberrant behaviour is that these two cellular processes, although occurring through similar initial pathways, arise due to the existence of a signalling bifurcation. Indeed, each of these receptors can transmit one signal eliciting cell death, and another that induces proliferation. This dichotomic signalization is dictated by the nature of the effector molecules recruited by the receptors, and the final cellular output depends on the relative frequency of these two signalization events. Thus, ISRAA is capable of binding to different receptors for the same or different activities, resulting either in a cell activation or death.

In one arrangement, the present invention concerns the therapeutic applications associated with the activity of the ISRAA polypeptide and related regulatory agents such as for use in a method of treating of cancer, neuromuscular disorders and diseases. More specifically such therapeutic applications concern methods of treating the following known diseases and disorders: cancer, Alzheimers Disease, Parkinson's disease, Amyotrophic Lateral Sclerosis (ALS) and Muscular dystrophies.

Immunogenic portions of the protein of the invention can be used to prepare antibodies specific for the proteins. Antibodies can be prepared which bind to an epitope in a region of the protein. The term antibody is also intended to include fragments which are specifically reactive with a protein, or peptide thereof, of ISRAA. Antibodies can be fragmented using conventional techniques, for example, F(ab')2 fragments can be generated by treating antibody with pepsin. The resulting F(ab')2 fragment can be treated to reduce disulfide bridges to produce Fab' fragments. Polyclonal antibodies are antibodies that are derived from different B-cell lines and are a mixture of immunoglobulin molecules secreted against a specific antigen, each recognising a different epitope. Monoclonal antibodies are antibodies that are identical because they were produced by one type of B-cell and are all clones of a single parent cell. Standard techniques are used to produce polyclonal antibodies. Routine procedure based on the hybridoma technique originally developed by Kohler and Milstein (Nature 256,495497 (1975)) is used to produce monoclonal antibodies. "Antibody fragments" comprise a portion of an intact antibody, preferably the antigen binding or variable region of the intact antibody. Examples of antibody fragments include Fab, Fab', F(ab')2, and Fv fragments; diabodies; linear antibodies (Zapata et al., Protein Eng. 8(10):1057-1062 [1995]); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, a designation reflecting the ability to crystallize readily. Pepsin treatment yields an F(ab')2 fragment that has two antigen-combining sites and is still capable of cross-linking antigen.

"Fv" is the minimum antibody fragment which contains a complete antigen-recognition and -binding site. This region consists of a dimer of one heavy-and one light-chain variable domain in tight, non-covalent association. It is in this configuration that the three CDRs of each variable domain interact to define an antigen-binding site on the surface of the VH-VL dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CHI) of the heavy chain. Fab fragments differ from Fab' fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa and lambda, based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA, and IgA2. "Single-chain Fv" or "sFv" antibody fragments comprise the VH and VL domains of antibody, wherein these domains are present in a single polypeptide chain. Preferably, the Fv polypeptide further comprises a polypeptide linker between the VH and VL domains which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994). An "isolated" antibody is one, which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

The word "label" when used herein refers to a detectable compound or composition which is conjugated directly or indirectly to the antibody so as to generate a "labeled" antibody. The label may be detectable by itself (e.g. radioisotope labels or fluorescent labels) or, in the case of an enzymatic label, may catalyze chemical alteration of a substrate compound or composition which is detectable.

The antibodies for use in accordance with the present invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')2 or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and coworkers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of ahumanantibody. Accordingly, such "humanized" antibodies are chimeric antibodies (U.S. Pat. No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol, 227:381 (1991); Marks et al, J. Mol. Biol, 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al, Bio/Technology 10, 779-783 (1992); Lonberg et al, Nature 368 856-859 (1994); Morrison, Nature 368, 812-13 (1994); Fishwild et al, Nature Biotechnology 14, 845-51 (1996); Neuberger, Nature Biotechnology 14, 826 (1996); Lonberg and Huszar, Intern. Rev. Immunol. 13 65-93 (1995).

A pharmaceutical composition comprising the isolated polypeptide having the amino acid sequence of SEQ ID NO: 3, for use in the treatment of: a) cancer; b) neurological diseases and disorders; or c) muscular diseases and disorders; wherein (i) the effective dose for treatment of cancer is at a concentration of 50 $\mu$g/ml; and (ii) the effective dose for treatment of neurological diseases and disorders or muscular diseases and disorders is at a concentration of 500 pg/ml is also within the scope of the present invention.

A pharmaceutical composition can be administered to a subject in an appropriate carrier or diluent, co-administered with enzyme inhibitors or in an appropriate carrier such as liposomes. The term "pharmaceutically acceptable carrier" as used herein is intended to include diluents such as saline and aqueous buffer solutions.

The therapeutic agent or pharmaceutical composition may be administered by a wide variety of routes. Exemplary routes of administration include oral, parenteral, transdermal, and pulmonary administration. For example, the therapeutic agent or composition may be administered intranasally, intramuscularly, subcutaneously, intraperitonealy, intravaginally and any combination thereof. For pulmonary administration nebulizers, inhalers or aerosol dispensers may be used to deliver the therapeutic agent or composition in an appropriate formulation (i. e., with anaerolizing agent). In addition, the therapeutic agent may be administered alone or in combination with other agents or known drugs and adjuvants. In combination, agents may be administered simultaneously or each agent may be administered at different times. When combined with one or more known drugs, agents and drugs may be administered simultaneously or the agent can be administered before or after the drug(s).

In one embodiment, the agents are administered in a pharmaceutically acceptable carrier. Any suitable carrier known in the art may be used. Carriers that efficiently solubilise the agents are preferred. Carriers include, but are not limited to a solid, liquid or a mixture of a solid and a liquid. The carriers may take the form of capsules, tablets, pills, powders, lozenges, suspensions, emulsions or syrups. The carriers may include substances that act as flavoring agents, lubricants, solubilizers, suspending agents, binders, stabilizers, tablet disintegrating agents and encapsulating materials.

Tablets for systemic oral administration may include excipients, as known in the art, such as calcium carbonate, sodium carbonate, sugars (e. g., lactose, sucrose, mannitol, sorbitol), celluloses (e. g., methyl cellulose, sodium carboxymethyl cellulose), gums (e. g., arabic, tragacanth), together with disintegrating agents, such as maize, starch or alginic acid, binding agents, such as gelatin, collagen or acacia and lubricating agents, such as magnesium stearate, stearic acid or talc.

In powders, the carrier may be a finely divided solid, which is mixed with an effective amount of a finely divided agent. In solutions, suspensions or syrups, an effective amount of the agent may be dissolved or suspended in a carrier such as sterile water or an organic solvent, such as aqueous propylene glycol. Other compositions can be made by dispersing the polypeptide agent or the inhibitor in an aqueous starch or sodium carboxymethyl cellulose solution, or in suitable oil known in the art.

The present invention will be further described with reference to the following examples that illustrate the embodiments of the invention with further reference to the following figures.

Figure 1 is a graphic representation illustrating detection of ISRAA in naive cells to which splenocyte supernatants from Trypanosoma-infected rats have been added.

Figure 2 is a graphic representation illustrating the effects of splenic denervation on induction of ISRAA

Figure 3 is a graphic representation illustrating the role of ISRAA in the modulation of innate responses and disease suppression.

Figure 4 is a graphic representation illustrating the effect of rISRAA on Peripheral Blood Mononuclear Cells (PBMCs).

Figure 5 is a graphic representation illustrating the cytotoxic effect of rISRAA on human Peripheral Blood Mononuclear Cells human (hPBMCs).

Figure 6 presents results from immunostating analysis of proliferation marker Ki-67 in PBMC cells treted with rISRAA.

Figure 7 is a graphic representation illustrating results of quantitative sandwich enzyme immunoassay to detect IFN-y in cells treated with 500 pg/ml rISRAA.

Figure 8 presents results from intracellular staining for detection of IFN-$\gamma$ expression in hPBMCs by Immunocytochemistry in cells treated with rISRAA.

Figure 9 is a graphic representation illustrating results from immunophenotyping analysis showing percentage of CD4 and CD8 cell types in cells treated with rISRAA.

Figure 10 is a graphic representation illustrating results from immunophenotyping analysis showing percentage of CD3, CD19 and $CD3^-$CD56 cell types in cells treated with rISRAA.

Figure 11 is a graphic representation illustrating results from immunophenotyping analysis showing percentage of CD4 (T Helper), CD8 (T Cytotoxic), CD19 (B Cells) and CD56 (NK cells) cell types in cells treated with rISRAA.

Figures 12 - 19 are graphic representations illustrating the effect of rISRAA on immunosuppressed cells in blood samples from eight kidney transplanted patients:

(a) results from dose kinetics study in eight patients
(b) cells proliferation activity in eight patients

Figure 20 presents results obtained by in situ cell death assay for study of cytotoxic effect on cells treated with 50 $\mu$g/ml of rISRAA.

Figure 21 is a photographic representation of immunohistochemistry stating of proliferative marker Ki-67 in patient's samples treated with rISRAA.

Figure 22 is a photographic representation of the effect of rISRAA on U-973 tumor cell line culture.

Figure 23 is a graphic representation illustrating results from cell death assay with U-973 tumor cell line treated with different concentrations of rISRAA.

Figure 24 is a photographic representation of the results of in situ cell death assay with U-973 tumor cell line treated with rISRAA.

Figure 25 is a graphic representation illustrating results from cell death assay in Kelly cell line treated with different concentrations of rISRAA.

Figure 26 is a photographic representation of the results of in situ cell death assay with Kelly cell line treated with rISRAA.

Figure 27 is a photographic presentation of the results of Papanicolaou Stain of rISRAA treated cells.

Figure 28 is a photographic presentation of the results of Giemsa Stain of rISRAA treated cells.

Figure 29 shows photographs of 14 days cultures of peripheral blood stem cells treated with rISRAA, leading to differentiation into nerve and muscle cells.

Figure 30 is a photographic presentation of Immunostaining of CD68 marker in cells treated with rISRAA.

Figures 31 and 32 shows photographs of 2 days cultures of embrionic brain cells treated with rISRAA, leading to differentiation into NF- or GFAP- positive nerve and glial cells.

The results shown in Figures 1-3 of the present specification provide the basis for the therapeutic potential of the ISRAA protein encoded by the *israa* gene. The experiments carried out to achieve these results are outlined in Examples 1-7 below and are summarised in the following paragraphs.

A population of Sprague-Dawley rats was injected subcutaneously with *T.b.brucei* parasites. Spleens were collected immediately after inoculation (less than one minute) and splenocytes were prepared. The splenocytes from these inoculated rats were cultured for 48 hours and thereafter supernatants were collected and tested for their ability to:

- induce naive rat cells to produce IFN-$\gamma$ (Figure 1A);
- induce naive rat cells to proliferate, as measured by $^3$H-thymidine-incorporation (Figure 1C).

A population of mice (Balb/c strain) was injected subcutaneously with infective stationary-phase promastigote *L. major* parasites, whilst a control group of mice did not receive any inoculum. Spleens were collected immediately after inoculation (less than one minute) and splenocytes were prepared. The splenocytes from the mice that had been inoculated with *L. major* parasites were cultured for 48 hours and thereafter supernatants were collected and tested for their ability to:

- induce naive mouse cells to produce TGF-$\beta$ (Figure 1B).

The presence of ISRAA was demonstrated using standard IFN-$\gamma$ immunoassays and $^3$H-thymidine-incorporation assays. Splenocyte supernatants from the trypanosoma infected rats stimulated naive cells to produce IFN-$\gamma$ (Figure 1A) and also to proliferate (as measured by $^3$H-thymidine-incorporation) (Figure 1C).
Detection of ISRAA was also demonstrated by intracellular detection of TGF-$\beta$ by immunohistochemistry (Figure 1B).

To investigate the involvement of the splenic nerve in the induction of ISRAA, splenocyte supernatants collected from denervated rat spleens challenged subcutaneously with *T.b.brucei* for less than one minute and cultured for 48 hours did not show biological activity as no induction of IFN-$\gamma$ was registered (Figure 2). Supernatants from sham-operated rats showed similar activity as those from non-denervated rats.

Further, the role of ISRAA in modulation of innate immune responses and disease suppression is depicted in Figure 3. Immunosuppression is a known feature of late disease of African trypanosomiasis where cells obtained 3 weeks post infections failed to proliferate when stimulated with con-A. However, addition of ISRAA to con-A-stimulated splenocytes obtained at week 3 after infection resulted in a significant proliferative response ($p<0.01$ when ISRAA added alone and $p<0.0001$ when ISRAA added with con-A), but no significant proliferative response was recorded with IL-2 compared to non-stimulated cultures (Figure 3).

**Example 1: Animals used**

In the EAT mode, Sprague-Dawley rats (Alab, Stockholm) aged 2 months and weighing about 200g were used, while Balb/c mice strain aged 2 months and weighing about 25g (supplied by the Animal Breeding Facility at the Faculty of Medicine of Arabian Gulf University - Kingdom of Bahrain) were used in the *L.major* model. The animals were housed 5 per cage, given food and water *ad libidum,* and maintained on a 12/12 hour light/dark cycle until sacrifice.

**Example 2: Surgical sympathectomy**

Only rats (Sprague-Dawley) where used here. The rats were weighed before surgery. Under pentobarbital sodium anesthesia (50 mg/kg), the fur on the operating area was shaved and the skin was sterilized with 70 % alcohol. A cut (5mm in length) was made on the skin and muscle by layers to expose the abdominal cavity. The ligamentum gastrosplenicum was cut. Since the celiac nerve runs along the celiac artery which arises from the abdominal aorta, the celiac artery was traced back to its origin by following its branches to the spleen. The celiac nerve branches off into the gastric nerve and the splenic nerve. The splenic nerve was isolated from the splenic vasculature and connective tissue near the bifurcation of the celiac artery and then the entire bundle of the nerve was cut. Immediately after surgery, each animal received an intraperitoneal injection of sulfadimethoxide (100 mg/rat) and then returned to the colony. Successful denervation was confirmed by histological studies. Sham-operated control rats were manipulated similarly, but without sympathectomy. The animals were recovered from surgery after 5 days, and one week later the infection was introduced.

**Example 3: Parasite strain and infection procedure**

The *T.b.brucei* strain, variable antigen type AnTat 1.1E isolated from bushbuck, was obtained from Dr. Nestor van Meirvenne, Laboratory of Serology, Institute of Tropical Medicine Prins Leopold, Antwerp, Belgium. Each rat (denervated or non-denervated sham-operated) was injected subcutaneously with 0.1 ml of a suspension of trypanosomes in a phosphate saline/glucose buffer, pH 8.0, containing about $10^6$ parasites per ml.

Promastigote stages of *L.major* parasites were grown in Medium 199 (M199; Life Technologies, Inc.) supplemented with 20% heat-inactivated fetal calf serum, 1M HEPES buffer (pH 7.4), penicillin /streptomycin (100X), L-glutamine (100X) (Gibco, Gaithersburg, Md.), 0.25% Hemin in 50% triethanolamine, 100mM adenine, 50mM Hepes, 0.1% Biotin in 95% ethanol (Sigma Aldrich, USA). The promastigote culture was maintained at 24-26°C. A 20% stock solution of Ficoll Type 400 (Sigma, St Louis, Mo) in sterile, endotoxin free water was prepared. Step gradients were prepared in which 2ml of 20% Ficoll at the bottom was overlaid by 2 ml of 10% Ficoll solution in Dulbecco's modified Eagles medium (DMEM) which was finally overlaid by 2ml of stationary-phase Promastigote ($2X10^8$/ml) suspended in DMEM. These step gradients were centrifuged for 15 minutes at 2500 rpm at room temperature. Metacyclics were collected from the top of interphase of DMEM 10% with Pasteur pipette. Infective stationary-phase promastigotes were collected by centrifugation, washed twice in ice-cold phosphate buffered saline, and resuspended at a density of $2x10^6$/ml. Parasites were inoculated in a volume of 50μl subcutaneous on the skin in experimental groups of mice while the control groups of mice didn't received any inoculum. Spleens were collected immediately after inoculation (less than a minute) and splenocytes were prepared (see below).

**Example 4: Preparation of splenocytes**

Spleens were dissected and crushed through a stainless steel meshwork. The cells were washed once in Dulbecco MOD Eagle Medium (Life Technology), supplemented with 2 mM L-glutamine (Flow), 50 IU penicillin (Astra, Södertälje, Sweden), 1% (vv) MEM (Flow), and 5% (vv) FCS (Gibco, Paisley, Scotland). Erythrocytes in the cell pellets were haemolysed by adding 2 ml cold water for 30 sec followed by addition of 1 ml 2.7% saline. The cells were then washed in medium twice and re-diluted to obtain a cell concentration of 5x $10^6$ ml and 200 μl aliquots were then applied to individual microtitre plate wells. There were no changes in the viability of the cells obtained from denervated or non-denervated rats. Splenocytes from animals that are inoculated with parasites were cultured for 48 hours and thereafter supernatants were collected and subjected to SDS electrophoresis or added to naive splenocytes and tested for their ability to induce the naive cells to IFN-γ and TGF-β production or to proliferation.

**Example 5: Single cell assay for IFN-γ production**

To test for ISRAA, the enzyme-linked immunospot (ELISPOT) assay was used (Mustafa et al 1991) to detect IFN-γ production by single secretory cells. In principle, nitrocellulose-bottomed 96 well microtitre plates (Millipore, Bedford, MA) were coated overnight with 100 μl aliquots of the mouse monoclonal antibody DB1, which is specific for IFN-γ at a concentration of 15 μg /ml (Van der Meide et al 1986). After repeated washings with PBS, 2% bovine serum albumin was applied for 2-4 hours, the plates were washed in PBS and splenocyte suspensions were applied followed by stimulation with splenocyte culture supernatants obtained from denervated and non-denervated rats inoculated with *T.b.brucei*. Some control cells were either stimulated with con-A or kept without stimulation. After incubation overnight at 37°C in humidified atmosphere of 7% $CO_2$, cells were removed by flicking the plate, followed by repeated washings in PBS. Polyclonal rabbit anti-rat IFN-γ (12), diluted 1/1000, was applied for 4 hours. After washing, biotinylated goat anti-rabbit IgG (Vector Lab, Burlingame, CA) was applied for 4 hours followed by an avidin-biotin-peroxidase complex (ABC Vectastain Elite Kit, Vector Lab). Colour development with 3-amino-9-ethylcarbazole and $H_2O_2$ was performed. Spots corresponding to cells that had secreted IFN-γ were counted using a dissection microscope.

**Example 6: Intracellular detection of TGF-β by immunohistochemistry**

In principle, immunostaining was performed as previously described (Sander et al 1991, Lore et al 1998). Briefly, cultured naive splenocytes were harvested after stimulation with splenocyte culture supernatants obtained from mice inoculated with *L.major*. The cells were washed in PBS and transferred to adhesion slides (BioRad Lab, Munich, Germany). Cells were allowed to adhere to the slides for 30 minutes at 37°C. Excessive cells were washed away. Cell fixation was performed in 2% formaldehyde in PBS at pH 7.4 for 10 minutes. The cells were then stored at -20°C until required for further investigation or directly processed for TGF-β detection. Endogenous peroxidase was blocked with 1 % $H_2O_2$ in 1x Earl's balanced salt solution (BSS) (Gibco) supplemented with 0.01 M HEPES buffer (Gibco) and 0.1% saponin (Riedel-de Haen, Seelze, Germany) for 30 minutes at room temperature. In order to reduce risks for non-specific antibody and hydrophobic interactions, the following precautions were undertaken: incubation with 2% FBS for 5 minutes at 37°C followed by incubation with 1% normal mouse sera for 30 minutes at 37°C. Additional incubation with blocking kit (Vector Laboratories, Burlingame, CA) was performed to block endogenous biotin or biotin-binding proteins. Cells were permeabilized with 0.1% saponin dissolved in BSS to allow the intracellular access of the cytokine-specific antibody. A total of 30 μl cytokine specific monoclonal antibody (mAb) (mouse anti-human TGF-β; R&D, Oxon, UK) diluted in BSS-saponin to a final concentration of 5 μg/ml was added and allowed to incubate for 30 minutes at 37°C followed by several washes in BSS. Non-specific staining by the second-step biotinylated goat antibody caused by Fc-interactions was prevented by a subsequent incubation with 1% goat serum (Dako, Glostrup, Denmark) dissolved in BSS-saponin for 15 minutes at room temperature. The biotin-conjugated secondary antibodies were then added. Goat anti-mouse IgG1 (Caltag Lab, San Francisco, CA) was used at 1/600 dilution in BSS-saponin. After three additional BSS washes, the cells were incubated with an avidin-biotin horse-radish peroxidase complex (Vectastain, Vector Laboratories) for 30 minutes at room temperature. A colour reaction was developed by 3'-diaminobenzidine tetrahydrochloride (DAB) (Vector Laboratories) and stopped after 2-10 minutes in the dark by washes in BSS. The cells were counterstained with hematoxylin and the slides were left to dry before mounting in buffered glycerol. The immunocytochemically stained cells were examined in a Leica RXM microscope (Leica, Wetzlar, Germany) equipped with a 3CDD colour camera (Sony, Tokyo, Japan). Enumeration of cytokine producing cells was performed manually at X 630 original magnification. The frequency of cytokine expressing cells was assessed by examination of at least 10.000 cells.

**Example 7: $^3$H-Thymidine-incorporation assay**

200 μl of splenocyte suspension ($5\times10^6$/ ml medium) was applied to each well of a round-bottom polystyrene 96-well microtitre plate (Nunclon, Nunc, Roskilde, Denmark). Six wells received either splenocyte culture supernatants obtained from denervated and non-denervated rats inoculated with *T.b.brucei,* or con-A at a final concentration of 5 μg/ml or no stimulation. In certain experiments 5 ng of ISRAA or rIL-2 were added either alone or each together with con-A to splenocytes obtained at late time points during the infection. The cells were incubated for 72 hours. Ten hours before harvest 10 μl aliquots containing 1 μCi of [$^3$H]-methylthymidine (specific activity 42 Ci/mmol) (Amersham, Little Chalfont, UK) in saline were added to each well. Cells were harvested onto glass fiber filters with a multiple channel semi-automated harvesting device (Titertek, Skatron AS, Lierbyen, Norway) and thymidine incorporation was measured as counts per minute (CPM) in a liquid beta-scintillation counter (Mark II, Searle Analytic, Des Plaines, IL, USA).

**Example 8: Effects of rISRAA on Peripheral Blood Mononuclear Cells (PBMCs)**

Human PBMCs from healthy donors were treated with different concentrations of rISRAA ranging from 5μg to 1pg per 1 x $10^5$ cells in 100μl of complete medium in each well, and cultured for 24, 48, and 72hrs. Then, cell proliferation, apoptosis, IFN-γ expression, quantitative sandwich enzyme immunoassay to detect IFN- γ, flow-cytometry and proliferation marker Ki-67 human PBMCs were monitored.

**Cell proliferation**

Cell proliferation: was not affected significantly by time in culture but significant difference was observed with different concentrations of rISRAA. Cells treated with 500pg/ml rISRAA (Figure 4 a,b) resulted in significantly more cell proliferation than in cells treated with 50μg/ml of rISRAA.

Figure 4 (a) presents the effects of rISRAA on Peripheral Blood Mononuclear Cells (PBMCs): (a) PBMCs from healthy donors (100)μl of 1x$10^6$cells/ml) were treated with different concentartion of rISRAA (50μg-10pg)/ml and proliferation of cells was monitored by CCK-80 assay. Cells treated with 50pg showed significant proliferation compared to those treated with 5μg. PHA was used as positive control while cells alone as negative control. Mann-Whitney's test was used to calculate the level of significance (*P<0.05, **P<0.005, ***P<0.0005.

Figure 4 (b) presents photographs (20X magnification) captured by inverted microscope showeing the proliferative response of hPBMCs under the effect of 50μg /ml and 500pg/ml rISRAA. PHA was used as a positive control and non-treated cells as a negative control respectively.

**Apoptosis and Cytotoxic Effect:**

The cytotoxic effect of rISRAA on human PBMCs (1x$10^5$/100μl culture medium) was determined by an *in situ* cell death assay in which 50μg/ml of rISRAA showed more apoptosis of the cells compared to 50pg/100μl of rISRAA and to the positive control which was treated with DNase (Figure 5).

Cytotoxic effect of 5μg of rISRAA on hPBMCs was monitored by in the situ cell death assay in which cleavage of genomic DNA during apoptosis yields double strand as well as single strand breaks ("nicks"), and then identified by labelling the free 3'-OH end with fluorescein. Cells treated with 5μg showed apoptosis of the cells (Figure 5b) comparing to the effect of the 50 pg of rISRAA on hPBMCs (Figure 5c) and to the positive control treated with DNase enzyme (Figure 5a), while in negative control the cells were incubated only in the presence of label solution without terminal transferase (Figure 5d).

**Proliferation Marker Ki-67:**

Staining and detection of the proliferation marker Ki-67 in both, cells stimulated with 50μg/ml or with 500pg/ml, showed higher expression of Ki-67 marker in cells treated with 500pg/ml (Figure 6 b) compared to cells treated with 50μg/ml of rISRAA (Figure 6a). Cells stimulated with 5μg/ml PHA was used as positive control (Figure 6c) and cells without treatment were used as negative control (Figure 6d).

Figure 6 presents the results of Ki-67 Immunostaining assay: (a) Ki-67immunostaining in cells treated with 50μg/ml rISRAA, showing nuclear negativity of cells compared to (b) Brown granular nuclear reactivity in cells treated with a 500 pg/ml (hematoxylin counterstain); (c) Positive control in which cells treated with 5μg/ml PHA and (d) negative control in which cells at resting stage were used (unstimulated PBMC). 20X and 40X magnification were used.

**Example 9: Quantitative sandwich enzyme immunoassay to detect IFN- γ**

To quantify and to analyze for expressed cytokine IFN-γ by human PBMCs which were treated with different concentrations of rISRAA, 50μg/ml, 500ng/ml and 500pg/ml; ELISA techniques with commercially available kits (Quantikine-Human IFN-γ Immunoassay) was used. Measurement of the absorbance at 450nm showed that cells treated with 500pg/ml rISRAA expressed more IFN-γ cytokines (62.5pg/ml) compared to cells treated with either 50μg or 500ng/ml of rISRAA. The amounts of IFN-γ released by cells were calculated by using a standard curve which was a plot of absorbance at 450 nm of different concentrations of IFN-γ (Figure 7 and Table 1).

Figure 7 represents a standard curve of IFN-γ cytokine concentration in cells treated with rISRAA, obtained by a sandwich ELIZA assay.

IFN-γ in cells treated with 500pg/ ml rISRAA was calculated from the standard curve. Cells stimulated with 5μg/ml PHA (positive control), and untreated cells were used as negative control.

Table 1: Measurements of the optical density of different samples treated with different concentration of rISRAA, PHA and without treatment at 450nm.

| Samples | Absorbance/450nm |
|---|---|
| cells alone ($1x10^6$/ml-Negative Control) | 0.08 20 |
| PHA(5µg/ml) (Positive Control) | 9.95 |
| ISRAA 50µg/ml | 0.07 |
| 500ng/ml | 0.06 |
| 500pg/ml | 0.20 |

**Example 10: Detection of IFN-γ Expression in Human PBMCs**

To examine the expression of IFN-γ in human cells treated with 50µg/ml and 500pg/ml of rISRAA, immunocytochemistry was used. Photographs of the treated cells were taken by digital camera fixed to a fluorescence microscope. These showed high expression of IFN-γ in cells treated with 500pg/ml ISRAA compared to 50µg/ml of rISRAA while PHA treated cells were used as positive control and untreated cells as negative control. In cells treated with 50µg/ml of rISRAA, few cells showed expression of IFN-γ compared to cells treated with 500pg/ml rISRAA, but they were larger and irregular in shape (Figure 8 a-d). Cells were treated with 50µg/ml rISRAA and 500pg/ml rISRAA and cultured for 24 hours then fixed and permeabilized. These were labelled with a primary antibody (mouse antihuman IFN-γ antibody) followed by a secondary antibody (Alexa Fluor 594 goat conjugated antimouse) then detected by fluorescence microscope.

Results from the intracellular staining of IFN-γ expression by Immunocytochemistry are presented on Figure 8: (a) expression of IFN-γ in healthy PBMCs treated with 50µg/ml rISRAA; (b) cells treated with 500pg/ml rISRAA and (c) positive control in which cells stimulated with 5µg/ml PHA and (d) negative control in which cells were grown alone without treatment. 20X and 40X magnification were used.

**Example 11: Flow Cytometry Assay**

By using immunophenotyping, specimens from healthy subjects were tested for the proportion of lymphocytes that are T cells, B cells, natural killer (NK) cells, $CD4^+$ T cells (helper T cells), and $CD8^+$ T cells (suppressor/inducer T cells). This was done by incubating anti-coagulated whole blood with monoclonal antibodies to the various cellular antigens that identify specific cell populations (phenotypes), and then lysing the red blood cells. The antibodies were conjugated to fluorescent tags that emit light of a certain wavelength when excited by a laser beam. The specimens were analyzed in a flow cytometer to determine the proportion of cells of a particular phenotype.

In flow cytometry assay the FACScan Becton Dickinson instrument and a double platform assay performed on a Beckman Coulter Epics Elite Instrument (CD45-FITC/CD4-RD1/CD8-ECD/CD3-PC5 Cod.6607013, and CD45-FITC/CD56-RD1/CD19-ECD/CD3-PC5 Cod.6607073 fully automated system) was used to examine the number and type of cell populations in whole blood which are stimulated by 50µg/ml and 500pg/ml of rISRAA.

In these experiments, CD3 identified T lymphocytes; CD8 identified suppressor/cytotoxic T lymphocytes, CD45 identified leucocytes and CD4 identified helper/inducer T lymphocyte which interact with class II molecules of the major histocompatibility complex (MHC). CD56 identified natural killer cells, while CD19 identified B cells.

Tables 2 and 3 show the data for all phenotypic cell markers on T, B or NK cells under the influence of 50µg/ml and 500pg/ml of rISRAA. There was no significant difference in either percentage or number of positive cells per microliter in either CD4 or CD8 as stimulated with 50µg/ml or 500pg/ml rISRAA (Figure 9 a - c). The expression of the B cell marker CD 19 was very low in both cell cultures treated with two different concentrations of rISRAA 50µg/ml and 500pg/ml (Figure 10 a and b).

Natural killer cells marker CD56 was significantly expressed in cells treated with 50µg/ml rISRAA compared to cells treated with 500pg/ml rISRAA (Figure 10c).

Table 2: Analysis data of CD45-FITC/CD4-RD1/CD8-ECD/CD3

| IDENTITY | 50μg/ml rISRAA | | | 500pg/ml rISRAA | | |
|---|---|---|---|---|---|---|
| | % | Cells/μl | Cell Number | % | Cells/μl | Cell Number |
| CD3 | 93.65 | 163 | 2316 | 95.97 | 171 | 2336 |
| CD3CD4 | 54.35 | 95 | 1344 | 154.68 | 97 | 1331 |
| CD3/CD8 | 37.4 | 65 | 927 | 39.81 | 71 | 969 |
| CD4/CD8 | 1.45 | | | 1.37 | | |

Table 3: Analysis Data of CD45-FITC/CD56-RD1/CD19-ECD/CD3-PC5

| IDENTITY | 50μg/ml rISRAA | | | 500pg/ml rISRAA | | |
|---|---|---|---|---|---|---|
| | % | Cells/μl | Cell Number | % | Cells/μl | Cell Number |
| CD3 | 93.02 | 164 | 2372 | 95.36 | 145 | 2119 |
| CD19 | 0.12 | 0 | 3 | 0.14 | 0 | 3 |
| CD3-CD56 | 6.39 | 11 | 163 | 3.69 | 6 | 82 |
| T+B+NK | 99.53 | | | 99.19 | | |

Figure 11 shows the overall expression of all markers on cells treated with 50μg/ml and 500pg/ml rISRAA, presented by percentage of CD4 (T Helper), CD8 (T cytotoxic), CD19 (B Cells) and CD56 (NK cells) in cells treated with 50μg/ml and 500pg/ml rISRAA.

**Example 12: Effects of rISRAA on immunosuppressed cells**

To examine the effect of rISRAA on immunosuppressed cells in blood samples obtained from eight kidney transplanted patients. The biological activity of rISRAA on immunosuppressed cells was monitored in terms of cell proliferation by *in situ* cell death and Proliferation Marker Ki-67 expression assays, and differential white blood cell counts. Dose kinetics study was examined for each patient. Results showed that cells proliferation were stimulated when treated with 500pg/ml rISRAA, while clear apoptosis was showed in cells treated with 50μg/ml rISRAA. PHA stimulated cells were used as positive control, while untreated cells were used as negative control in all experiments (Figures 12-19). Results from dose response study on PBMCs (a); and cells proliferation activity measured by MTT assay (b) in samples from eight patients are shown on Figures 12-19. P value was calculated by ANOVA in which *=P<0.05; **=P<0.05; ***=P<0.0005.

**Example 13: In situ Cell Death Assay**

To examine the cytotoxic effect of 50μg/ml rISRAA on patients' cells (immunosuppressed cells) as well as on healthy donor cells and comparing it with the proliferative effect of 500pg/ml of rISRAA on both cells, *in situ* cell death, fluorescence assay was performed. Figure 20 (a) shows apoptosis of the cells treated with 5μg/ml rISRAA compared to cells treated with 50pg/ml rISRAA which did not show any cell death (Figure 20b). These results were compared to the positive control cells treated with DNase (Figure 20c) and a negative control treated with buffer (Figure 20d). 20X and 40XMagnification were used.

**Example 14: Proliferation Marker Ki-67 expression**

Cell proliferation was studied by a detection of expression of Ki-67 proliferation marker, detected in cell culture samples from patients and healthy donors (control) treated with 50μg/ml and 500pg/ml rISRAA by using immunohistochemistry. Positive cells were stained brown while the negative cells did not stain. The detection was positive in cell samples from all patients, which were stimulated with 50pg/ml rISRAA, as well as in samples from healthy blood donors (Figure 21a), while it was negative in cells treated with 50μg/ml rISRAA (Figure 21b). Results were compared with a positive control in which cells were stimulated with PHA (Figure 21c) and a negative control in which cells were cultured alone without stimulation (Figure 21d). 20X and 40X magnifications were used for the photographs presented on Figure 21.

**Example 15: Effect of rISRAA on tumour cell lines**

To study the effect of rISRAA on tumour cell lines in terms of cell proliferation and cytotoxicity, MTT assay was used together with *in situ* Cell Death assay. Histiocytic lymphoma U-937 cell line and Kelly (Human neuroblastoma cell line) Nuclear Lysate cell line were used to examine the effect of different concentrations of rISRAA on cells death and proliferation. Dose and time responses studies were done for both cell lines. Cells were treated with different concentration of rISRAA ranging from 50μg-10pg/ml and incubated for 24, 48 and 72 hours at 37°C, 5%$CO_2$. MTT Cell Proliferation assay was used to study the cytotoxicity of rISRAA on both cell lines. Absorbance at 690nm was measured for each titer of rISRAA and the Cytocide rate was calculated from the below formula:

$$\text{Cytocide Rate (\%)} = (OD_{\text{Control}} - OD_{\text{Experiment}})/OD_{\text{Control}} \times 100.$$

Results showed that the death rate was the highest in cells treated with 50μg/ml rISRAA at all time intervals 24, 48, and 72 hours compared to the other concentrations of rISRAA in both cell lines. Furthermore, the results showed that in the U-937 cell line the highest rate of cell death occurred at 48 hours (97.63%) (Figure 23b) compared to the 24 hours rate (95.89%) (Figure 23a) and 72 hours (92.32%) (Figure 23c), while the cell death rate in Kelly cell line remained constant at different time intervals (66.67%) (Figure 25 a - c).

Death rate was also examined in cells treated with 50μg/ml and 500pg/ml of rISRAA and incubated for 48 hours by using *in situ* cell death assay in which the free 3'-OH terminal was labelled with fluorescein. Results showed high rate of apoptosis in cells treated with 50μg/ml of rISRAA (Figure 24a) compared to 500pg/ml (Figure 24b) and to negative control in which cells were untreated (Figure 24 d). DNase treated cells wwre used as positive control (Figure 24 c).

Cytotoxic effect of 50μg/ml rISRAA was examined by *in situ* cell death assay compared to the effect of 500pg/ml rISRAA on the Kelly cell line. Labeling of DNA strand breaks is carried out by TUNEL-reaction assay in which Terminal deoxynucleotidyl transferase (TdT) catalyzes incorporation of labeled nucleotides to the free 3'-OH end of DNA strand in a template-independent manner. Cells were treated with (a) 5μg rISRAA and (c) 50pg rISRAA and incubated for 24 hours at 37°C in 5% $CO_2$. Then, fixed in 4% paraformaldehyde, permeabilized in 0.1% sodium citrate with 0.1% Triton X-100 for 2 min on ice. 50μl of TUNEL reaction mixture (Label Solution plus Enzyme solution Terminal deoxynucleotidyl transferase-TdT) was added to cells treated with rISRAA, while in (d), negative control mixture, fixed and permeabilized cells were incubated in 50 μl/well Label Solution (Nucleotide mixture in reaction buffer without terminal transferase) instead of TUNEL reaction mixture. For positive control (b), fixed and permeabilized cells incubated with recombinant DNase I, grade I (3000 U/ml- 3 U/ml in 50 mM Tris-HCl, pH 7.5, 10 mM MgCl2 1 mg/ml BSA) for 10 min at 15-25°C to induce DNA strand breaks, prior to labeling procedures. Magnifications of 40X and 100X were used for the photographs.

Results showed that apoptosis was very significant in cells treated with 50μg/ml rISRAA compared to the apoptosis rate in cells treated with 500pg/ml rISRAA. All results were compared to positive control in which cells were treated with DNase, and with negative control in which cells were incubated with mixture of nucleotides in reaction buffer without terminal transferase (Figure 26 a - d).

**Example 16: Giemsa and Papanicolaou Staining**

To differentiate nuclear and/or cytoplasmic morphology of cells in most experiments with cell cultures (cells alone, cells with PHA and cells treated with 50μg/ml rISRAA and cells with 500pg/ml rISRAA), Giemsa and Papanicolaou stains were used. The photomicrographs presented on Figures 27 and 28 showed obvious differences between different treatments: cells treated with 500pg/ml rISRAA showed higher stimulation of the cells proliferation (Figure 27c) compared to cells stimulated with PHA (Figure 27b) and to non-stimulated cells (Figure 27a). In contrast, cells treated with 50μg/ml rISRAA, showed death of most cells and at the same time showed stimulation of low number large cells, containing apoptotic bodies in its cytoplasm (Figure 27d). Magnifications of 40X and 100X were used for the photographs on Figure 27 presenting the Papanicolaou Stained cells treated with different concentration of rISRAA. Magnifications of 20X, 40X and 100X were used for the Figure 28 photographs of Giemsa stained cells treated with different concentration of rISRAA.

**Example 17: Differentiation of peripheral blood stem cells**

Cells treated with rISRAA (50μg/ml and 500pg/ml of culture media) were incubated for 15 days at 37°C, 5% $CO_2$ to monitor any changes in cell growth. Cells stimulated with PHA and also cells cultured without stimulation were disintegrated and lysed while cells which were treated with 5μg and 50pg of rISRAA per 100ul cell culture, showed growth of different types of cells with different shapes and sizes as shown on Figure 29 presenting photographs of 14 days cell cultures treated with rISRAA leading differentiation into nerve and muscle cells. Magnifications of 20X and 40X were used on Figure 29.

Examining the expression of CD68 marker on monocytes and macrophages further identified the cell-types. The results of the CD68 immunostaining assay presented on Figure 30, showed dark brown colour, indicating that these cells, which were also larger in size, were monocytes and macrophages, while the other spindle-shaped cells were not identified and were left for further investigation.

**Example 18: Differentiation of embryonic brain cells**

The role of ISRAA in stimulation of embryonic brain cells differentiation in glial and nerve cells was determined by immunohistochemistry using two cell-type specific antigenic markers glial fibrillary acidic protein (GFAB) and neurofilament (NF). Impregnated Balb/c mice at various stages of gestation, early (E 11), middle (E15), late periods (E 18) or postnatal were sacrificed by cervical dislocation at the end of each period. The uterus was removed and the embryos were separated from it. The brains of the embryos were then dissected. The isolated tissues were dissociated into single cells and diluted in Dulbecco's modified Eagle's medium/Ham's F12 nutrient mixture with the addition of 10% fetal calf serum (FCS). Cells were then plated in poly-d-lysine-coated 8-chamber glass slides and grown for 72 hours followed by developmental expression by immunohistochemistry.

Cells obtained from a balb/c mice at 15 days of gestation, were cultured for 2- 10 days in DMEM/F12 Ham media and were either treated with 50 pg or with 5 μg of ISRAA protein, or not treated at all. Results showing differentiation into NF- marker positive cells and GFAP-marker positive astrocytes after 2 days of culture in the presence of 50 pg of ISRAA are presented on Figures 31 and 32 respectively.

SEQ ID No:1

Full Length Sequence

5′
CCCAGTTAGTGCCTGAGTACGAGAGACTCATCAGGGCTGAGAAAGGAGAATGGCAGGGG
AAACAGTGCTACAAGGATGTCCTTTGTGTCCTGACCATGCAGAAGGAGACAGAAGCCCCA
GGATTGGTGGTTCCCAGAGGCAGAGCCCACTCCTGGAGGTGACTCACACTCAGTGTTGTC
ATTTGATCCTTAGCTGTCTGTATGTCTGGTGCCCTGTGACCCAGTCTCTTTATGGTGCTCCG
GAGCAGCATTCCTTTCTGTCCCCCCTTGTTCTTCTCCACGCTTCCCAACGACCAGGTACACA
CCCACTTGCCATCTCCCTGTTAACCCTACCTTCCCAGTTAGTGCCTGAGTACACAGAAGTG
ACATCTCTCCAGGTCATCAGAATTCTTGTCAATCCCTCTGAGTCAGCAAACTGCCTGGGTT
AGAAGTCTGGGACTTCTCTGGGACTCAGAACCCAATGCCCAGAATCTGAGCCACATCAAG
GCTGGTTGTTCTCCCTCTCCTCATCTTGAGAGTTTGGCTTGTGCATGACAGGCCTGCCTGCC
CACTTAGTTAGCCTTCCTTCCTTTCTTCCTAACTTCCTGATGGCCTTCGAAGCCCCTAGACT
TACCATATCACTACCAAAAGAAAAAAGAAAAAGAAAAAGCTGTAAGTGGGAAGTGACCC
ACTTTTGACCCCTGGTACATGCATCCCAGGTAGATGCCTTGGTCCCTTGTCTTATTGGAACT
GATTCTAGCTCATCCATGAGCTGAAGGAAGTCAGTCCTTTGACCCAAGCTTGGGACTGTGG
TGTCCTGGCTACATTATCTCCCTTCTCACCCCTCCTCTAGAGGGTCCTTCAGGGGGCGGGCT
CCCCACTGCCAGAGGATAAAGCATCCCCGACTCCGGAGCCAGCCAGCCATGCCACAGCAC
TGTATTTATTGGCGGCTCCTCTTCACTCAGGCCCACGACAGGAGTTCAGACCCCCGCGAGA
AAAGCCACCCGCTATTGATCCTTAGTCAGGTCTCCGGAGCCTGGCAGACATTCATCAAGG
AGCCACAGCATGGGGCTACAGCTCGCCCTTTCTTTCTTCTTCCTTTCTTCTACCCCCCAAGT
CATTCCCCCCCCCCGGGGGGGGGTCCGAGGGAGGCGGGTGAGGGGGCTTGCCGAGGTCAC
TCCAAGCACTGCGGGGTGGGGTGGGGTGGGGTGGGGTTGGCTGTGGGCCCCGGGCTTTTG
AGCAAGTCTTTGGAAGTTAGAGATGTGGAACTTGGAGCGAGTCAGGGAAAGCCCTTCTAA
CGAAGATAGTGTGTGGAGTGCCGGCATTTCTGTAGACTCCAATACTAAAAAGAGGCTGCT
AACCAAGAGGGCCTTGAGGGATTTGATTCCAGAGAAGAGAGCCTAGAAATTAGCAAGTG
AAAGGGGCCAGTTTGGACTGATGAATGGGTGCTTGAAAACAATTTGTTTTCTTAAGTATGT
AAATATGCTAGTGAAGAGTATTTACTTTTTGGGTTACCTTGGAAAACCCTGTATCTTGAAT
ATTATTTCCATCCTCAGGGTCCCACTCAGGAGCTTCTGAAGTGGGTCCCGAGGAGAACTAA
GAGGTAGGAGGACGAGGAGGCAAAACAACGGGAAGCTGTCTTTATAATTTTTAAAGGATT
ACCACCACCAAGTCCCTGCCCCTAAGGTGTAAACTTTGCTCAGTTCCAGCCTGAATGTTCA
CTCAACCTGCAGGAATGATCTTTTTTAATATATGTATTTCAAAATATATGTATATTTTATTT
ATATATGTATATATCATGTATGAATATGTTTGCTTGCTTATATGTCCGTGTTCCACTTGTGT
GTTTGGTGCCCTCGGAATAGAAGAGGGTGTTGAATTTCCCTAAACCTGGAGTTTCAGATGA
TTGTGAGCTGCCACATGGGTGCCAAGGAGAGAATTTTGGCCCTTTGCAAGAGCAAGAAAC
CTTTTTTAACCACTGAGCCATTTTTCCTGCCCCAGGACTTTTTTTGTAAAGTTAGTTCTTTTT
GCTACTGTGTGGGTAAGCCCTTGGCCACTGAACAGGCCCTATGAATCAGAAAAAAAAAAA
AAAAAAAAAAAAAAAAAAAAAAAAA

SEQ ID No:2

atggcaggggaaacagtgctacaaggatgtcctttgtgtcctgac

95 catgcagaaggagacagaagccccaggattggtggttcccagagg

140 cagagcccactcctggaggtgactcacactcagtgttgtcatttg

185 atccttagctgtctgtatgtctggtgccctgtgacccagtctctt

230 tatggtgctccggagcagcattcctttctgtccccccttgttctt

275 ctccacgcttcccaacgaccaggtacacacccacttgccatctcc

320 ctgttaaccctaccttcccagttagtgcctgagtacacagaagtg

365 acatctctccaggtcatcagaattcttgtcaatccctctgagtca

410 gcaaactgcctgggttag 427

SEQ ID No:3

M A G E T V L Q G C P L C P D
H A E G D R S P R I G G S Q R
Q S P L L E V T H T Q C C H L
I L S C L Y V W C P V T Q S L
Y G A P E Q H S F L S P L V L
L H A S Q R P G T H P L A I S
L L T L P S Q L V P E Y T E V
T S L Q V I R I L V N P S E S
A N C L G *

**Claims**

**1.** An isolated polypeptide molecule having the amino acid sequence of SEQ ID NO: 3, for use in the treatment of:

a) cancer;
b) neurological diseases and disorders; or
c) muscular diseases and disorders; wherein

(i) the effective dose for treatment of cancer is at a concentration of 50 μg/ml; and
(ii) the effective dose for treatment of neurological diseases and disorders or muscular diseases and disorders is at a concentration of 500 pg/ml.

**2.** A polypeptide for use according to claim 1, wherein said treatment of cancer is achieved by an apoptotic activity of the polypeptide on cancer cells; and wherein said treatment of neurological diseases and disorders is achieved by stimulation of the differentiation of peripheral blood stem cells or embryonic brain cells to nerve and glial cells; and wherein said treatment of muscular diseases and disorders is achieved by stimulation of the differentiation of peripheral blood stem cells to muscle cells.

**3.** A polypeptide for use according to either claim 1 or claim 2, wherein said neurological diseases and disorders, or muscular diseases and disorders are neuromuscular disorders such as Alzheimer's Disease, Parkinson's disease, Amyotrophic Lateral Sclerosis (ALS) and Muscular dystrophy.

**4.** A polypeptide for use according to claim 1, **characterised by** a potent dualistic activity which is:

(i) an apoptotic activity on tumour cells, or
(ii) a proliferative activity on normal immune cells and differentiation activity on peripheral blood stem cells and embryonic brain cells.

5. A pharmaceutical composition comprising a polypeptide molecule having the amino acid sequence of SEQ ID NO: 3, and any one or more pharmaceutically acceptable carriers, adjuvants, diluents or excipients, for use in the treatment of:

   a) cancer;
   b) neurological diseases and disorders; or
   c) muscular diseases and disorders; wherein

      (i) the effective dose for treatment of cancer is at a concentration of 50 μg/ml; and
      (ii) the effective dose for treatment of neurological diseases and disorders or muscular diseases and disorders is at a concentration of 500 pg/ml.

6. A pharmaceutical composition for use according to claim 5, wherein said treatment of cancer is achieved by an apoptotic activity of the polypeptide on cancer cells; and wherein said treatment of neurological diseases and disorders is achieved by stimulation of the differentiation of peripheral blood stem cells or embryonic brain cells to nerve and glial cells; and wherein said treatment of muscular diseases and disorders is achieved by stimulation of the differentiation of peripheral blood stem cells to muscle cells.

7. A pharmaceutical composition for use according to either claim 5 or claim 6, wherein said neurological diseases and disorders, or muscular diseases and disorders are neuromuscular disorders such as Alzheimer's Disease, Parkinson's disease, Amyotrophic Lateral Sclerosis (ALS) and Muscular dystrophy.

8. A pharmaceutical composition for use according to claim 5, **characterised by** a potent dualistic activity which is:

   (i) an apoptotic activity on tumour cells, or
   (ii) a proliferative activity on normal immune cells and differentiation activity on peripheral blood stem cells and embryonic brain cells.

9. A method for stimulating *in vitro* the differentiation of the peripheral blood stem cells into nerve and muscle cells, comprising contacting with or introducing into said cells a polypeptide molecule having the amino acid sequence of SEQ ID NO: 3; or a pharmaceutical composition comprising said polypeptide molecule.

10. A method for stimulating *in vitro* the differentiation of embryonic brain cells into nerve and glial cells, comprising contacting with or introducing into said cells a polypeptide molecule having the amino acid sequence of SEQ ID NO: 3; or a pharmaceutical composition comprising said polypeptide molecule.

## Patentansprüche

1. Isoliertes Polypeptidmolekül, das die Aminosäuresequenz SEQ ID NR: 3 aufweist, zur Verwendung bei der Behandlung von:

   a) Krebs;
   b) neurologischen Erkrankungen und Störungen; oder
   c) Muskelerkrankungen und -störungen; wobei

      (i) die wirksame Dosis zur Behandlung von Krebs bei einer Konzentration von 50 pg/ml liegt; und
      (ii) die wirksame Dosis zur Behandlung von neurologischen Erkrankungen und Störungen oder Muskelerkrankungen und -störungen bei einer Konzentration von 500 pg/ml liegt.

2. Polypeptid zur Verwendung nach Anspruch 1, wobei die Behandlung von Krebs durch eine apoptotische Wirkung des Polypeptids auf Krebszellen erzielt wird; und wobei die Behandlung von neurologischen Erkrankungen und Störungen durch Stimulation der Differenzierung von peripheren Blutstammzellen oder embryonischen Gehirnzellen in Nerven- und Gliazellen erzielt wird; und wobei die Behandlung von Muskelerkrankungen und -störungen durch Stimulation der Differenzierung von peripheren Blutstammzellen in Muskelzellen erzielt wird.

3. Polypeptid zur Verwendung nach Anspruch 1 oder Anspruch 2, wobei die neurologischen Erkrankungen und Störungen oder Muskelerkrankungen und -störungen neuromuskuläre Störungen wie Alzheimer-Krankheit, Parkinson-Krankheit, amyotrophe Lateralsklerose (ALS) und Muskeldystrophie sind.

4. Polypeptid zur Verwendung nach Anspruch 1, **gekennzeichnet durch** eine starke dualistische Wirkung, die Folgendes ist:

   (i) eine apoptotische Wirkung auf Tumorzellen oder
   (ii) eine proliferative Wirkung auf normale Immunzellen und eine Differenzierungswirkung auf periphere Blutstammzellen und embryonische Gehirnzellen.

5. Pharmazeutische Zusammensetzung, umfassend ein Polypeptidmolekül, das die Aminosäuresequenz von SEQ ID NR: 3 aufweist, und eine oder mehrere pharmazeutisch annehmbare Träger, Adjuvanzien, Verdünnungsmittel oder Hilfsstoffe zur Verwendung bei der Behandlung von

   a) Krebs;
   b) neurologischen Erkrankungen und Störungen; oder
   c) Muskelerkrankungen und -störungen; wobei

      (i) die wirksame Dosis zur Behandlung von Krebs bei einer Konzentration von 50 pg/ml liegt; und
      (ii) die wirksame Dosis zur Behandlung von neurologischen Erkrankungen und Störungen oder Muskelerkrankungen und -störungen bei einer Konzentration von 500 pg/ml liegt.

6. Pharmazeutische Zusammensetzung zur Verwendung nach Anspruch 5, wobei die Behandlung von Krebs durch eine apoptotische Wirkung des Polypeptids auf Krebszellen erzielt wird; und wobei die Behandlung von neurologischen Erkrankungen und Störungen durch Stimulation der Differenzierung von peripheren Blutstammzellen oder embryonischen Gehirnzellen in Nerven- und Gliazellen erzielt wird; und wobei die Behandlung von Muskelerkrankungen und -störungen durch Stimulation der Differenzierung von peripheren Blutstammzellen in Muskelzellen erzielt wird.

7. Pharmazeutische Zusammensetzung zur Verwendung nach Anspruch 5 oder Anspruch 6, wobei die neurologischen Erkrankungen und Störungen oder die Muskelerkrankungen und -störungen neuromuskuläre Störungen wie Alzheimer-Krankheit, Parkinson-Krankheit, amyotrophe Lateralsklerose (ALS) und Muskeldystrophie sind.

8. Pharmazeutische Zusammensetzung zur Verwendung nach Anspruch 5, **gekennzeichnet durch** eine starke dualistische Wirkung, die Folgendes ist:

   (i) eine apoptotische Wirkung auf Tumorzellen oder
   (ii) eine proliferative Wirkung auf normale Immunzellen und eine Differenzierungswirkung auf periphere Blutstammzellen und embryonische Gehirnzellen.

9. Verfahren zur In-vitro-Stimulation der Differenzierung der peripheren Blutstammzellen in Nerven- und Muskelzellen, umfassend das Inkontaktbringen mit den oder Einführen in die Zellen eines Polypeptidmoleküls, das die Aminosäuresequenz von SEQ ID NO: 3 aufweist; oder eine pharmazeutische Zusammensetzung, umfassend das Polypeptidmolekül.

10. Verfahren zur In-vitro-Stimulation der Differenzierung von embryonischen Gehirnzellen in Nerven- und Gliazellen, umfassend das Inkontaktbringen mit den oder Einführen in die Zellen eines Polypeptidmoleküls, das eine Aminosäuresequenz von SEQ ID NR: 3 aufweist; oder eine pharmazeutische Zusammensetzung, umfassend das Polypeptidmolekül.

**Revendications**

1. Molécule de polypeptide isolée ayant la séquence d'acides aminés de SEQ ID N° 3, destinée à être utilisée dans le traitement :

   a) du cancer ;

b) de maladies et troubles neurologiques ; ou
c) de maladies et troubles musculaires ; dans laquelle

(i) la dose efficace pour le traitement de cancer est à une concentration de 50 pg/ml ; et
(ii) la dose efficace pour le traitement de maladies et troubles neurologiques ou de maladies et troubles musculaires est à une concentration de 500 pg/ml.

**2.** Polypeptide destiné à être utilisé selon la revendication 1, dans lequel ledit traitement du cancer est obtenu par une activité apoptotique du polypeptide sur les cellules cancéreuses ; et dans lequel ledit traitement de maladies et troubles neurologiques est obtenu par stimulation de la différenciation de cellules souches du sang périphérique ou de cellules cérébrales embryonnaires en cellules nerveuses et gliales ; et dans lequel ledit traitement de maladies et troubles musculaires est obtenu par stimulation de la différenciation de cellules souches du sang périphérique en cellules musculaires.

**3.** Polypeptide destiné à être utilisé soit selon la revendication 1, soit selon la revendication 2, dans lequel lesdites maladies et troubles neurologiques ou lesdites maladies et troubles musculaires sont des troubles neuromusculaires tels que la maladie d'Alzheimer, la maladie de Parkinson, la sclérose latérale amyotrophique (SLA) et la dystrophie musculaire.

**4.** Polypeptide destiné à être utilisé selon la revendication 1, **caractérisé par** une activité duale puissante qui est :

(i) une activité apoptotique sur les cellules tumorales, ou
(ii) une activité proliférative sur les cellules normales immunitaires et une activité de différenciation sur les cellules souches du sang périphérique et les cellules cérébrales embryonnaires.

**5.** Composition pharmaceutique comprenant une molécule de polypeptide ayant la séquence d'acides aminés de SEQ ID N° 3, et l'un quelconque ou plusieurs parmi des véhicules, adjuvants, diluants ou excipients pharmaceutiquement acceptables, destinée à être utilisée dans le traitement :

a) du cancer ;
b) de maladies et troubles neurologiques ; ou
c) de maladies et troubles musculaires ; dans laquelle

(i) la dose efficace pour le traitement du cancer est à une concentration de 50 pg/ml ; et
(ii) la dose efficace pour le traitement de maladies et troubles neurologiques ou de maladies et troubles musculaires est à une concentration de 500 pg/ml.

**6.** Composition pharmaceutique destinée à être utilisée selon la revendication 5, dans laquelle ledit traitement du cancer est obtenu par une activité apoptotique du polypeptide sur les cellules cancéreuses ; et dans laquelle ledit traitement de maladies et troubles neurologiques est obtenu par stimulation de la différenciation de cellules souches du sang périphérique ou de cellules cérébrales embryonnaires en cellules nerveuses et gliales ; et dans laquelle ledit traitement de maladies et troubles musculaires est obtenu par stimulation de la différenciation de cellules souches du sang périphérique en cellules musculaires.

**7.** Composition pharmaceutique destinée à être utilisée soit selon la revendication 5, soit selon la revendication 6, dans laquelle lesdites maladies et troubles neurologiques ou lesdites maladies et troubles musculaires sont des troubles neuromusculaires tels que la maladie d'Alzheimer, la maladie de Parkinson, la sclérose latérale amyotrophique (SLA) et la dystrophie musculaire.

**8.** Composition pharmaceutique destinée à être utilisée selon la revendication 5, **caractérisée par** une activité duale puissante qui est :

(i) une activité apoptotique sur les cellules tumorales, ou
(ii) une activité proliférative sur les cellules immunitaires normales et une activité de différenciation sur les cellules souches du sang périphérique et sur les cellules cérébrales embryonnaires.

**9.** Procédé de stimulation *in vitro* de la différenciation de cellules souches du sang périphérique en cellules nerveuses et musculaires, comprenant la mise en contact avec ou l'introduction dans lesdites cellules d'une molécule de polypeptide ayant la séquence d'acides aminés de SEQ ID N° 3 ; ou d'une composition pharmaceutique comprenant ladite molécule de polypeptide.

**10.** Procédé de stimulation *in vitro* de la différenciation de cellules cérébrales embryonnaires en cellules nerveuses et gliales, comprenant la mise en contact avec ou l'introduction dans lesdites cellules d'une molécule de polypeptide ayant la séquence d'acides aminés de SEQ ID N° 3 ; ou d'une composition pharmaceutique comprenant ladite molécule de polypeptide.

**Figure 1:**

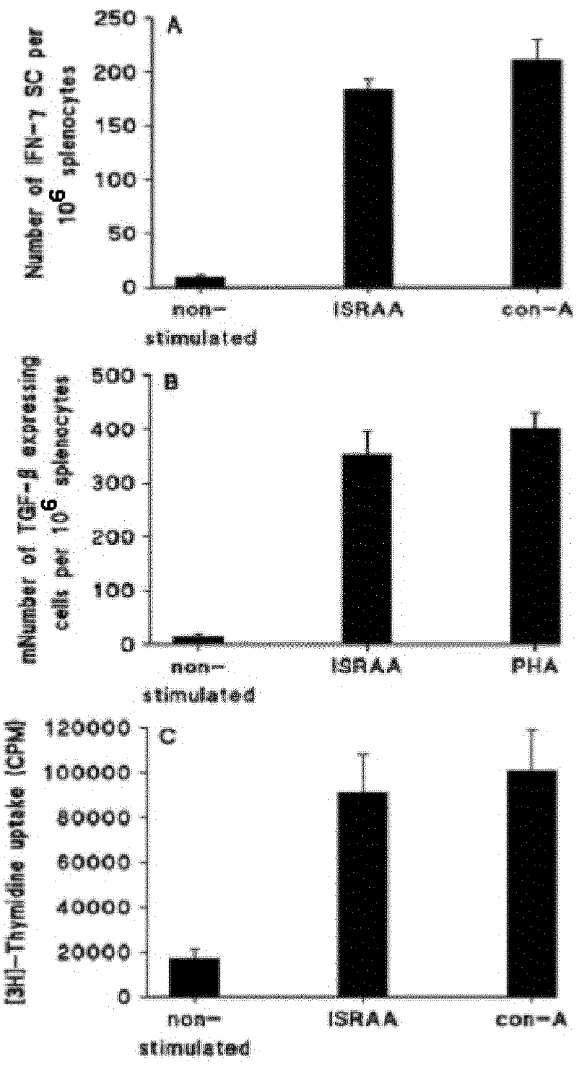
A
Number of IFN-γ SC per 10^6 splenocytes
250
200
150
100
50
0
non-stimulated
ISRAA
con-A
B
mNumber of TGF-β expressing cells per 10^6 splenocytes
500
400
300
200
100
0
non-stimulated
ISRAA
PHA
C
[3H]-Thymidine uptake (CPM)
120000
100000
80000
60000
40000
20000
0
non-stimulated
ISRAA
con-A

Figure 2

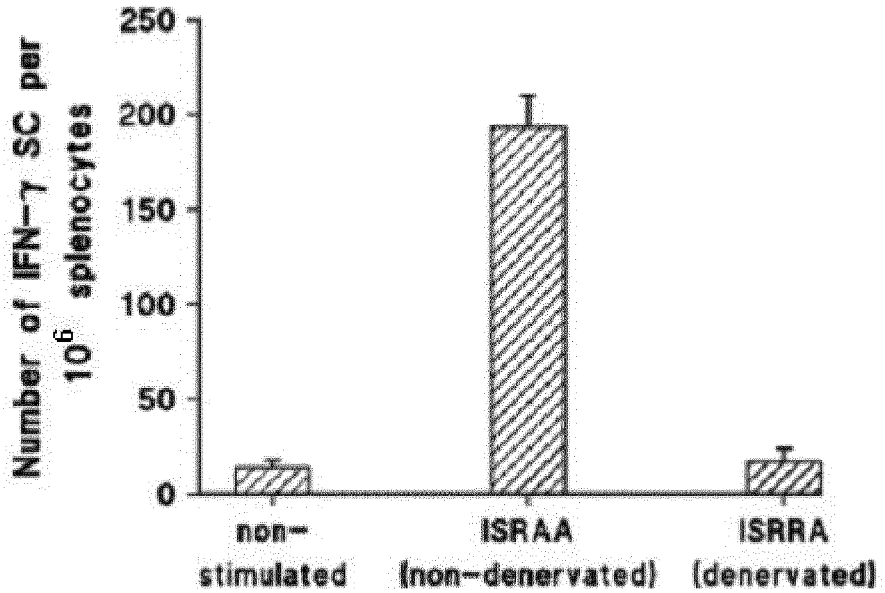
Number of IFN-γ SC per 10^6 splenocytes
250
200
150
100
50
0
non-
stimulated
ISRAA
(non-denervated)
ISRRA
(denervated)

Figure 3

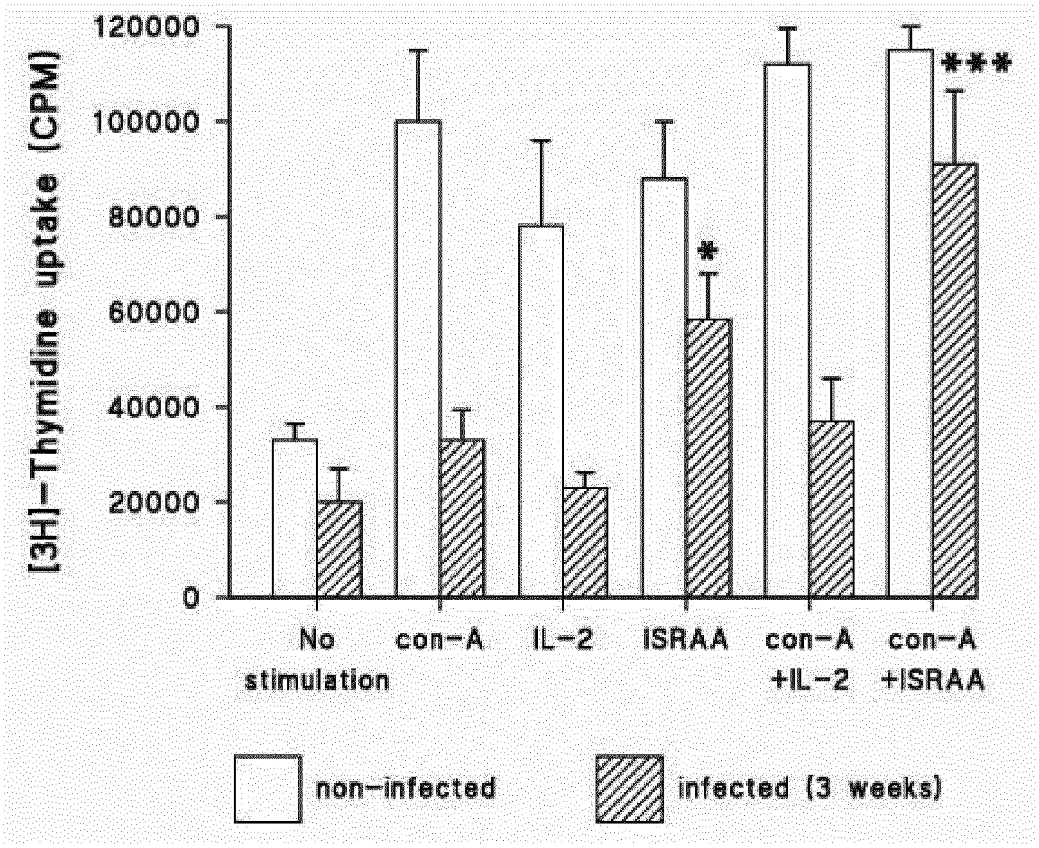
[3H]-Thymidine uptake (CPM)
120000
100000
80000
60000
40000
20000
0
*
***
No stimulation
con-A
IL-2
ISRAA
con-A +IL-2
con-A +ISRAA
non-infected
infected (3 weeks)

**Figure 4: Effects of rISRAA on Peripheral Blood Mononuclear Cells (PBMCs).**

(a)

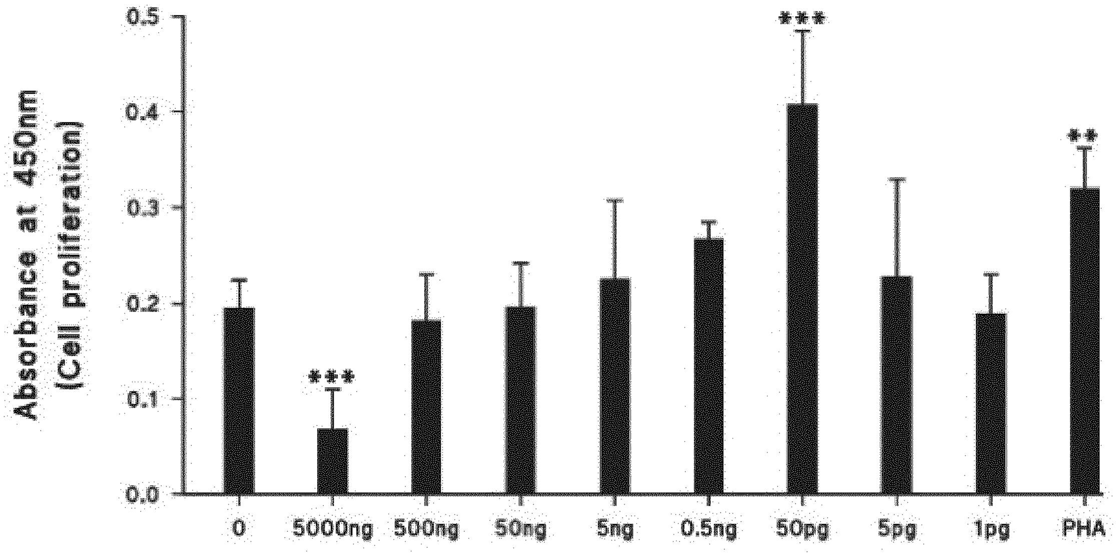


(b)

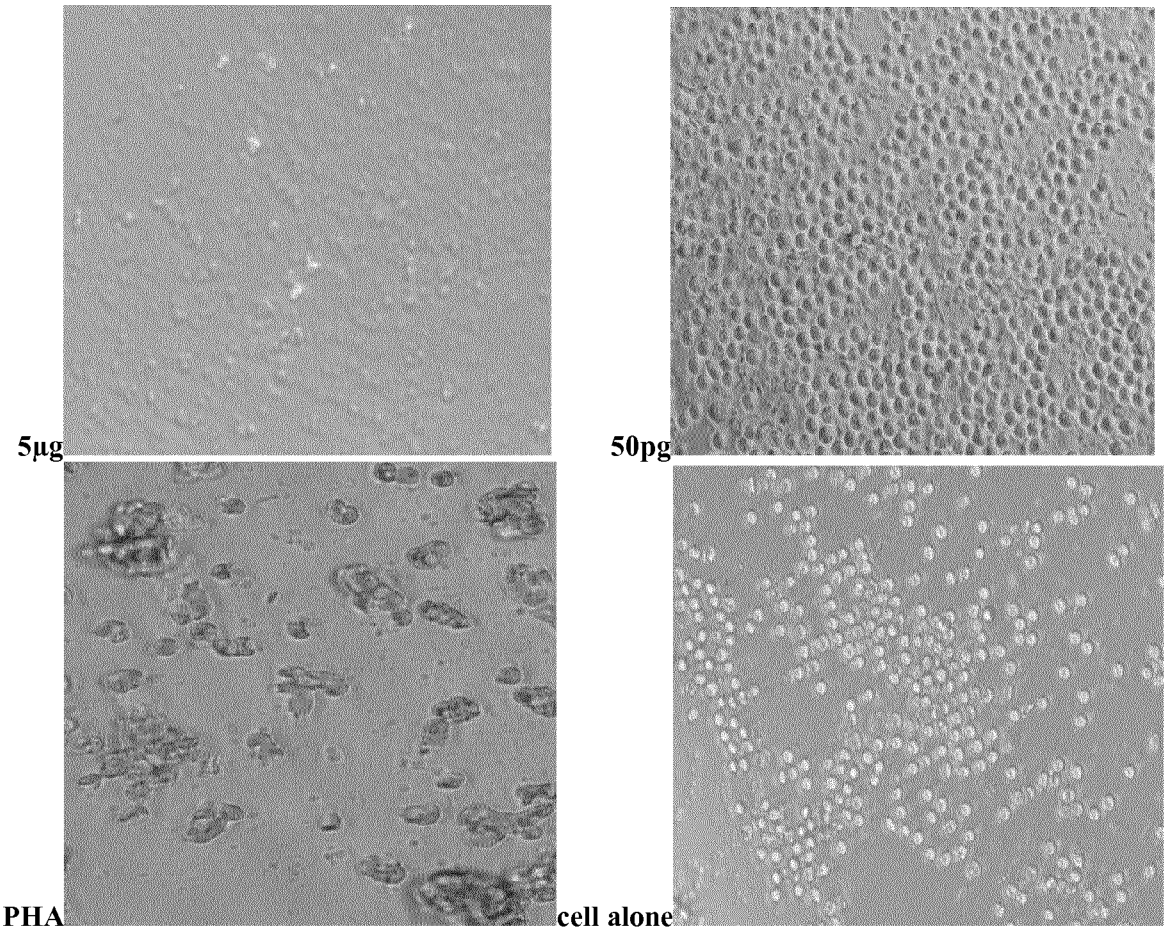

**Figure 5: Cytotoxic effect of rISRAA on hPBMCs.**

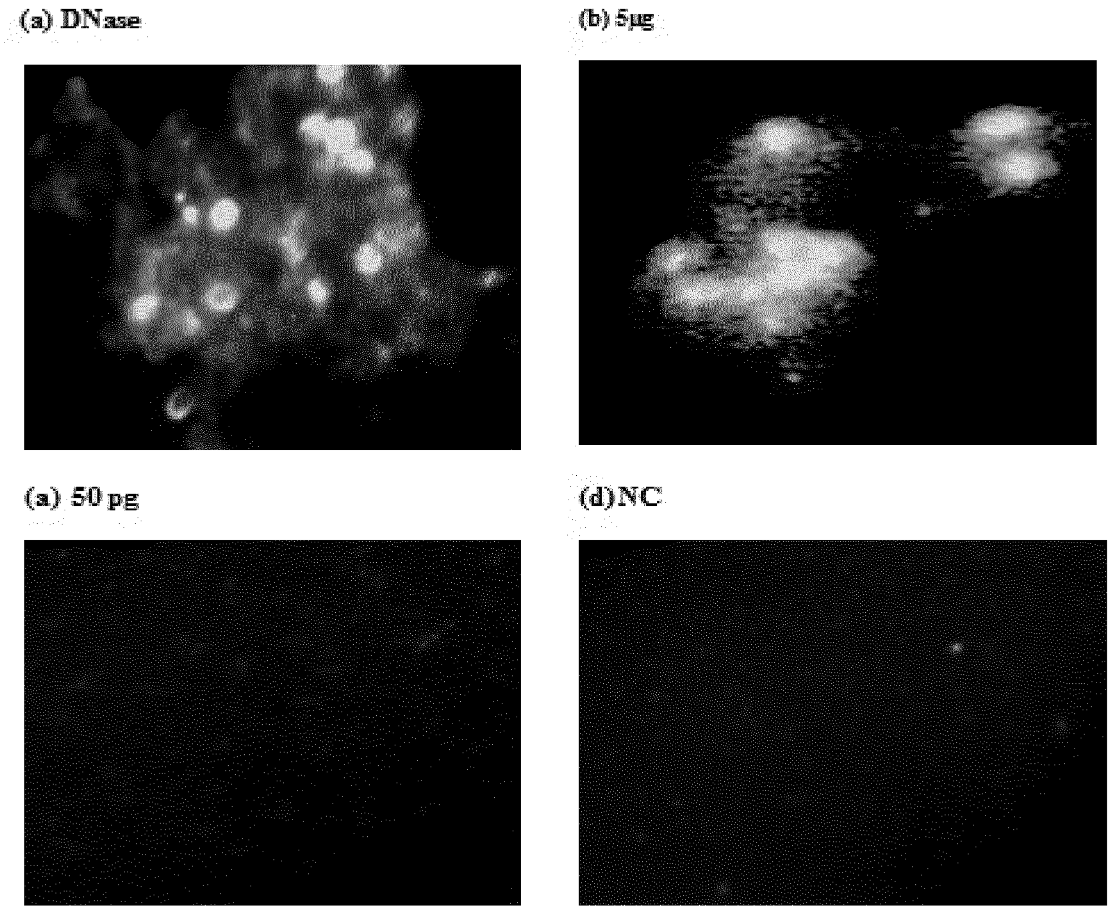

**Figure 6: Ki-67 Immunostaining**

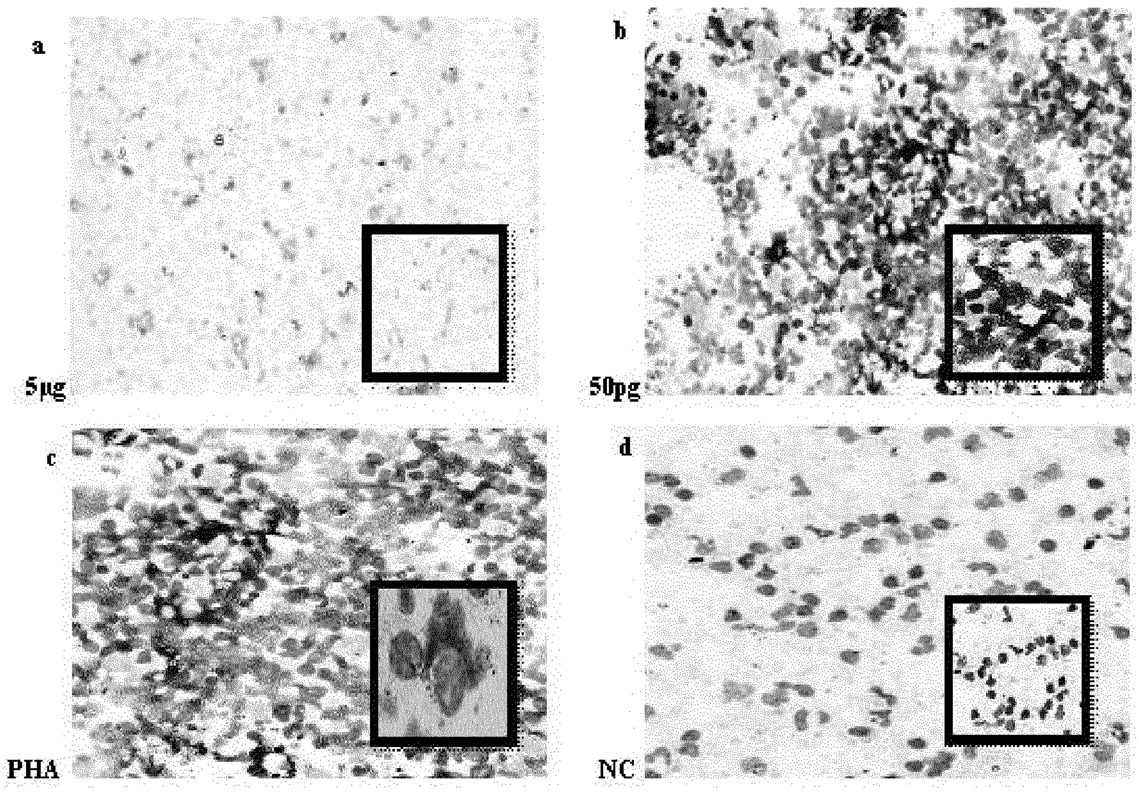

Figure 7: Standard curve of IFN-γ cytokine using a sandwich ELIZA assay.

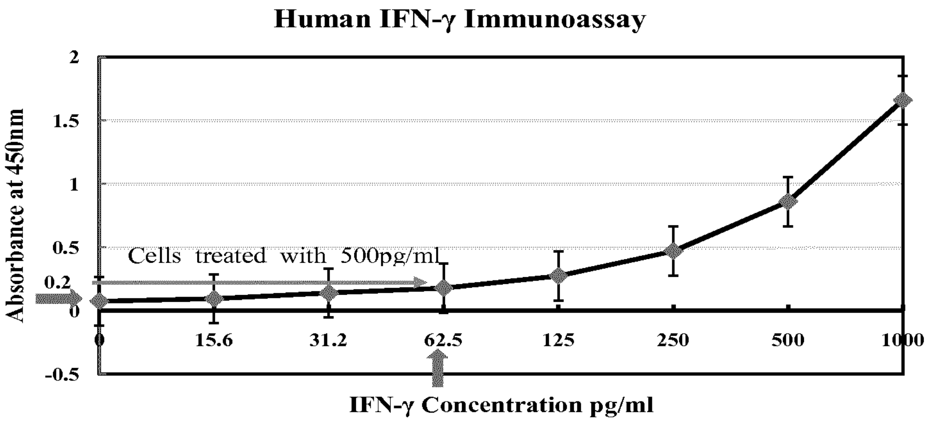


Figure 8: Intracellular staining of IFN-γ expression by Immunocytochemistry.

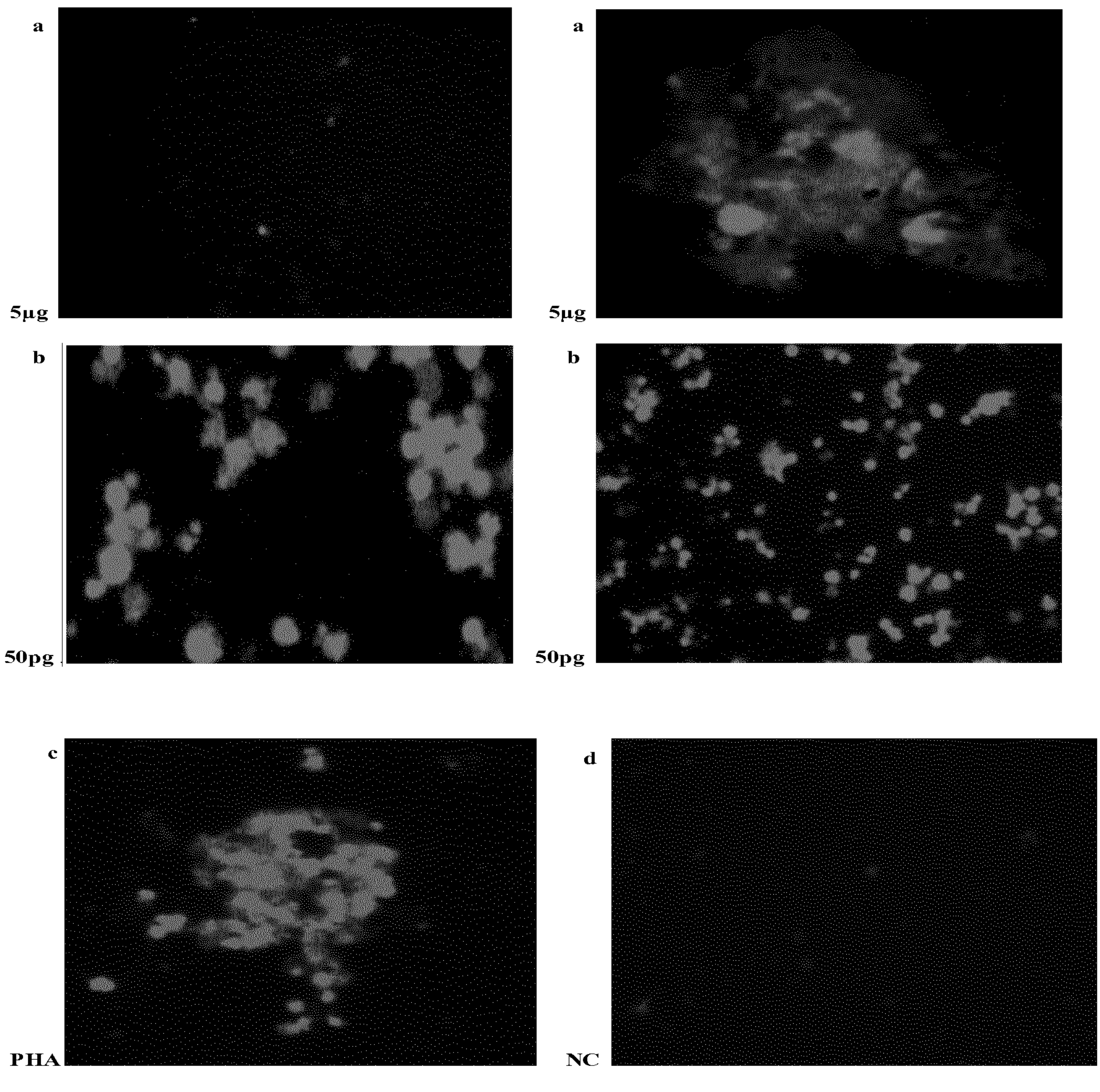

Figure 9: Percentage of cell Types CD4 and CD8.

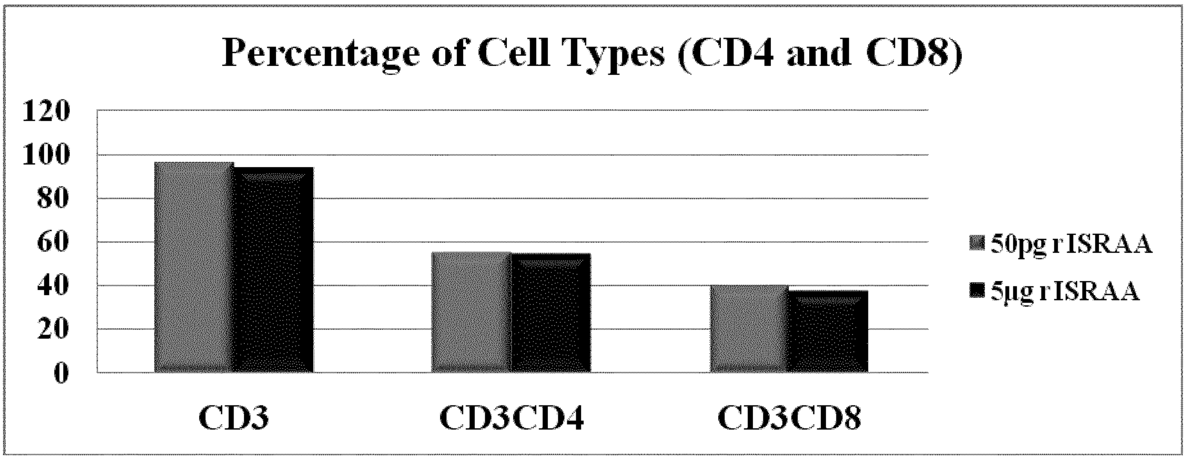


(a) Percentage of cell Types CD4 and CD8 in cells treated with 50pg and 5μg of rISRAA.

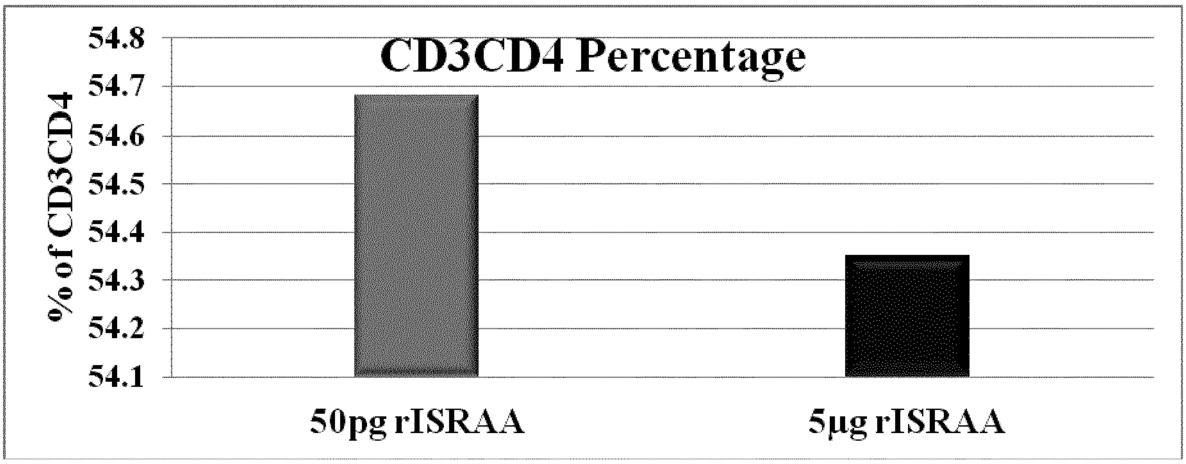


(b)Percentage of CD3CD4 in Cells treated with 5μg and 50pg rISRAA

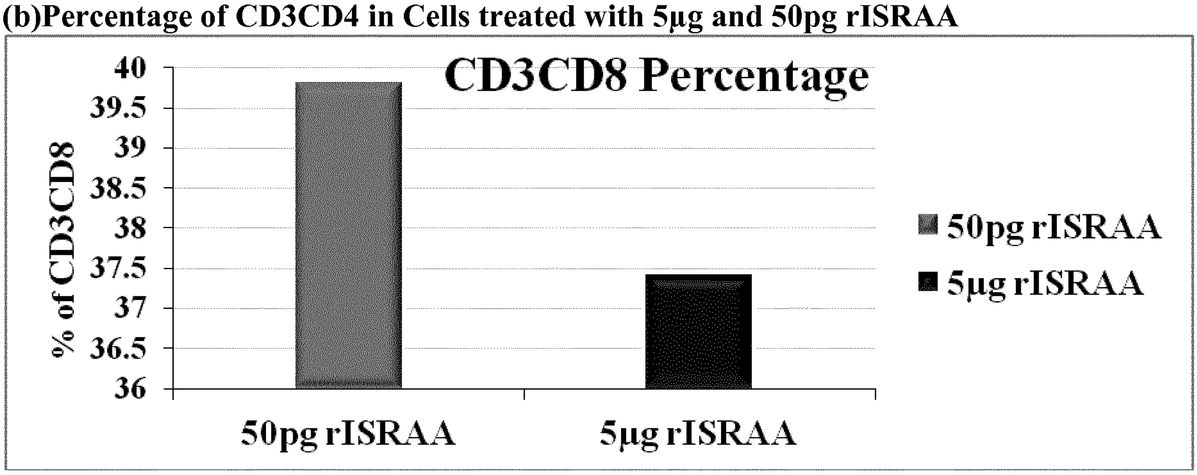


(c)Percentage of CD3CD8 in Cells treated with 5μg and 50pg of rISRAA

Figure 10: Percentage of cell types CD3,CD19, and $CD3^{-}CD56$.

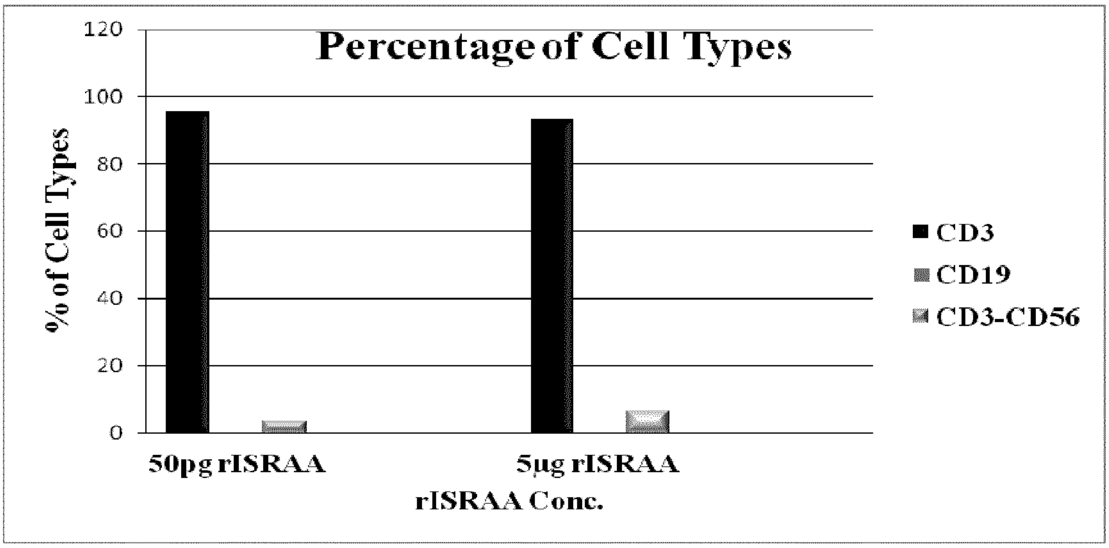


(a) Percentage of cell types CD3,CD19, and $CD3^{-}CD56$ in cells treated with 5µg and 50pg of rISRAA

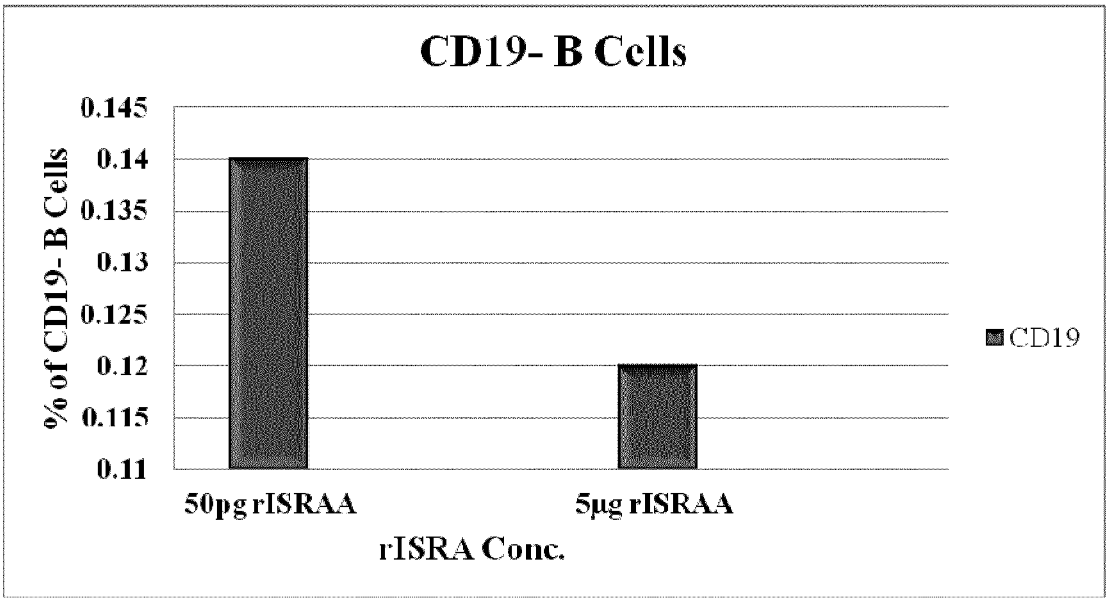


(b) Percentage of CD19-B Cells marker in Cells treated with 5µg and 50pg rISRAA.

**Figure 10: Percentage of cell types CD3,CD19, and CD3⁻CD56.**

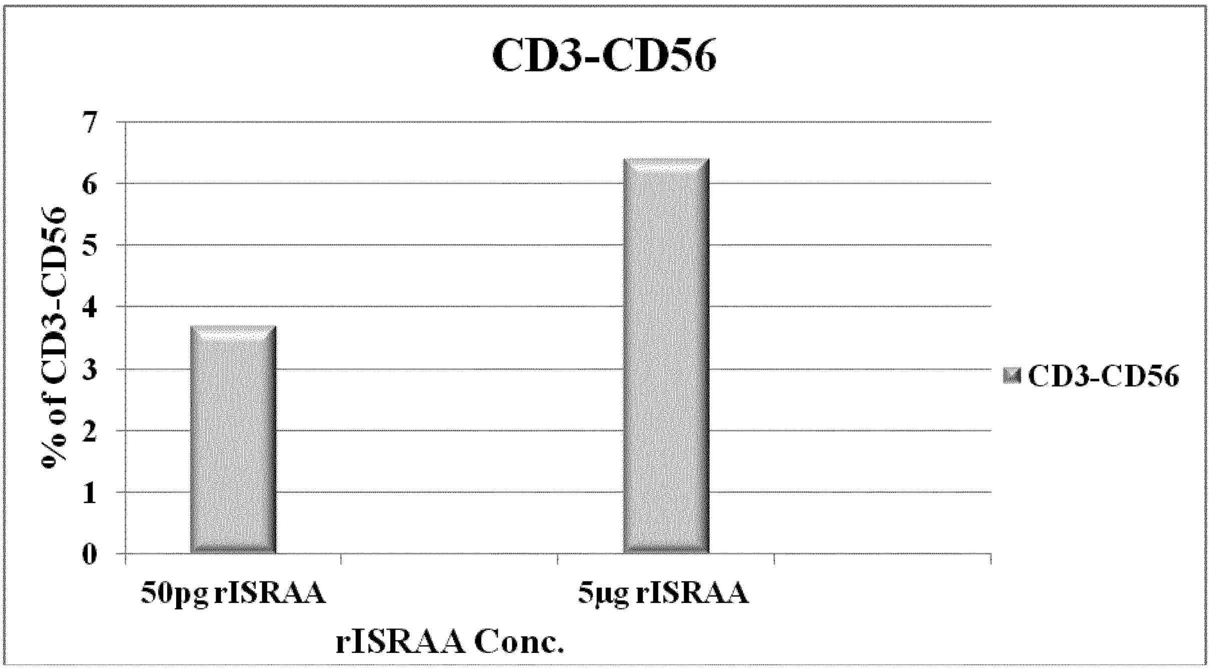


**(c) Percentage of CD56 Natural killer cells marker in cells treated with 5µg/ml and 50pg/ml of rISRAA**

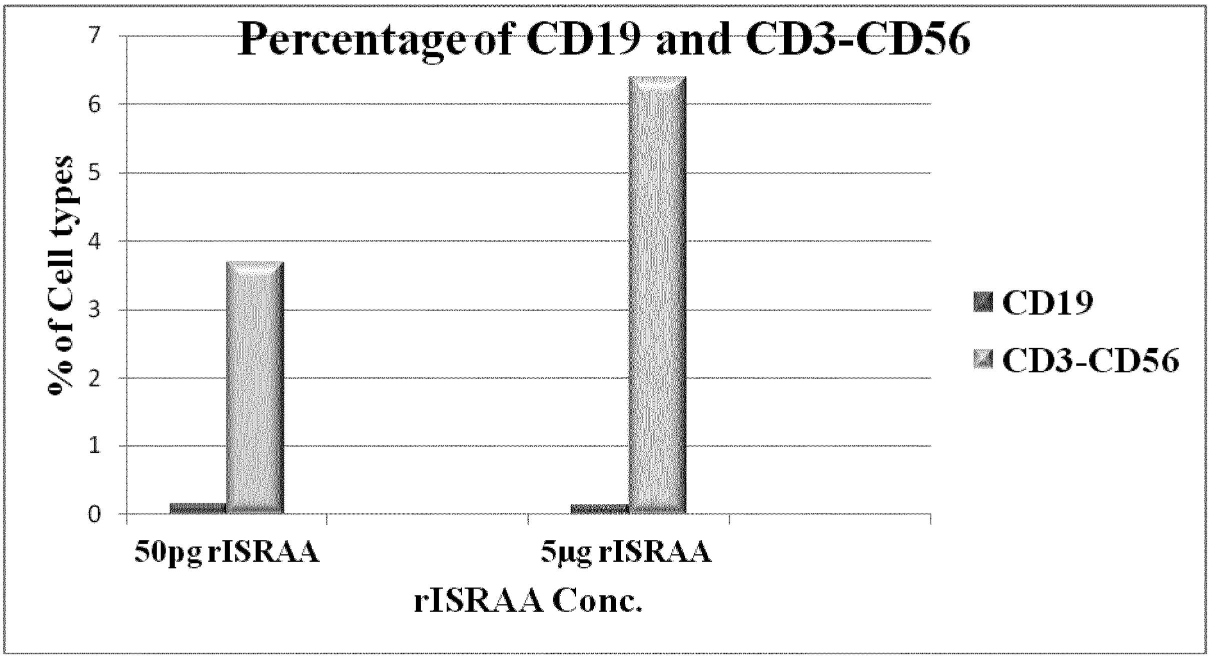


**(d) Comparison of the percentage of CD19 and CD56 in cells treated with 5µg/ml and 50pg/ml of rISRAA**

**Figure 11: Percentage of CD4 (T Helper), CD8 (T Cytotoxic), CD19 ( B Cells) and CD56 (NK cells).**

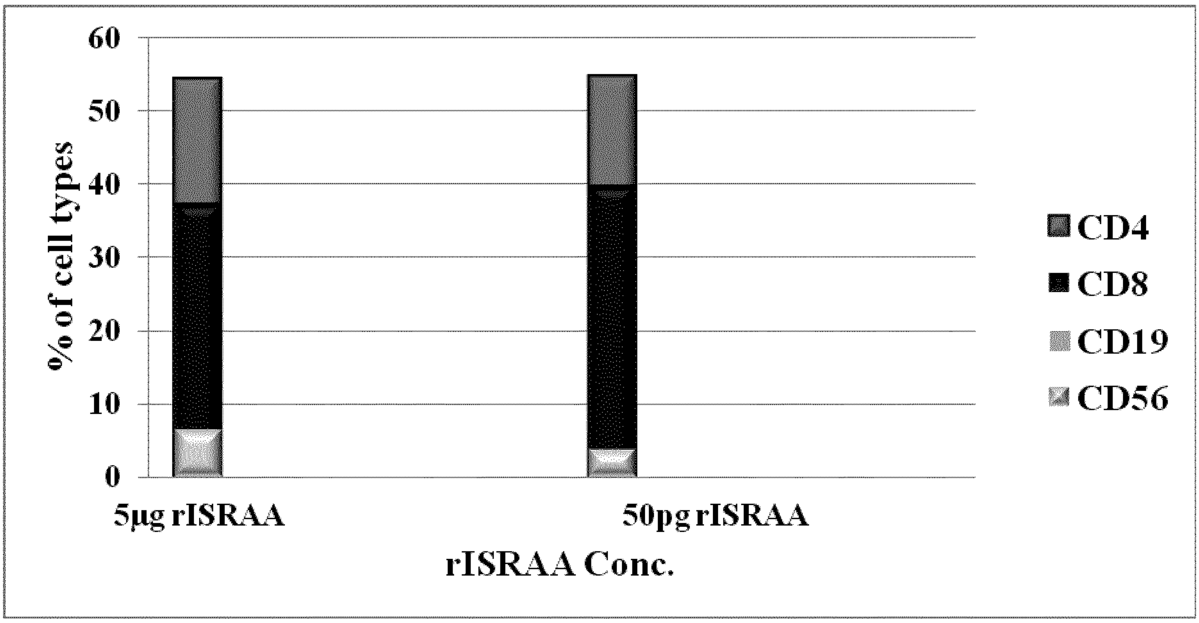

Figure 12: Effect of rISRAA on Immunosuppressed Cells - Patient 1

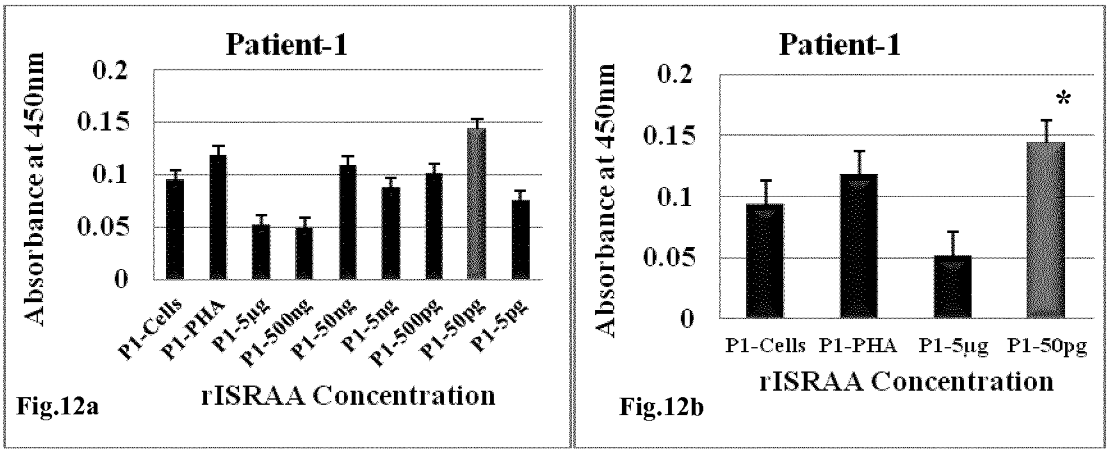


Figure 13: Effect of rISRAA on Immunosuppressed Cells - Patient 2

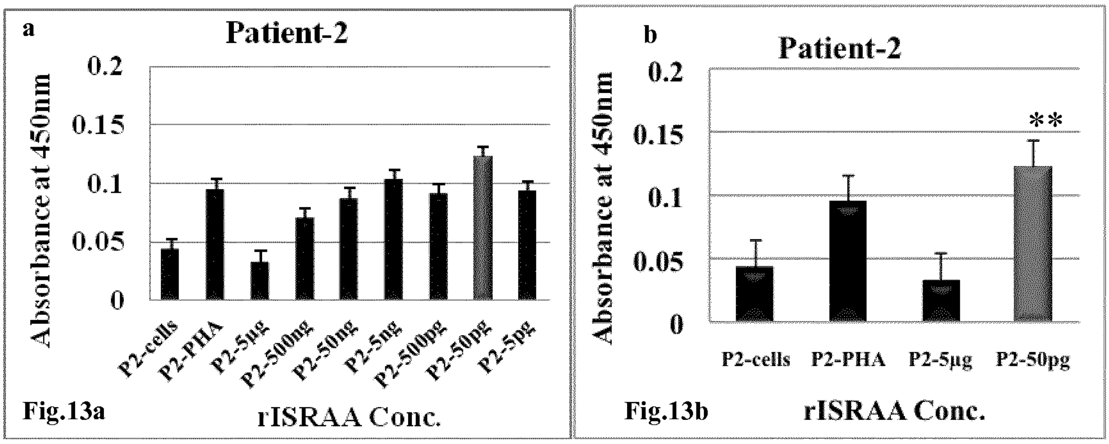


Figure 14 : Effect of rISRAA on Immunosuppressed Cells - Patient 3

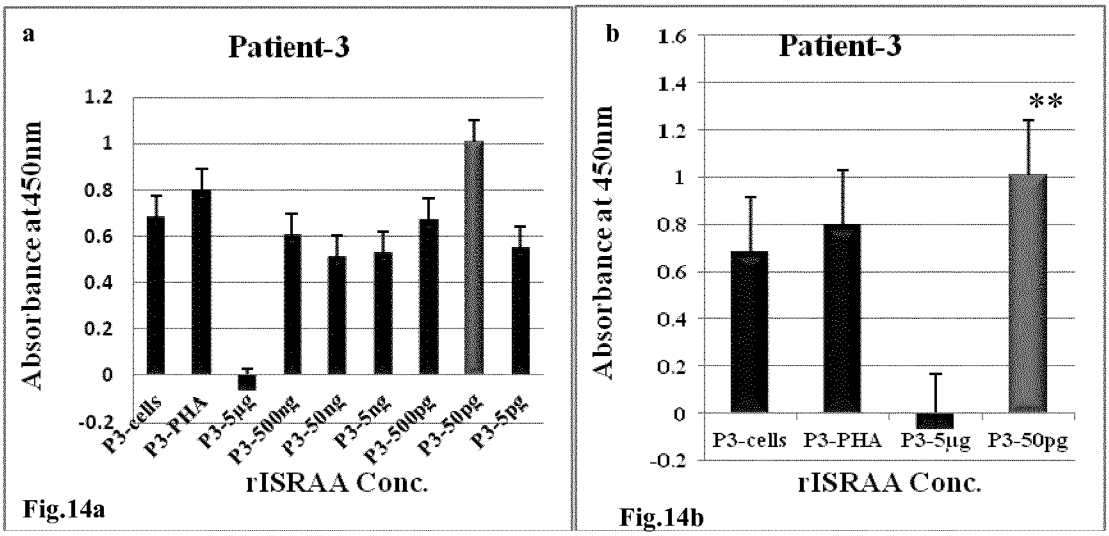

**Figure 15: Effect of rISRAA on Immunosuppressed Cells - Patient 4**

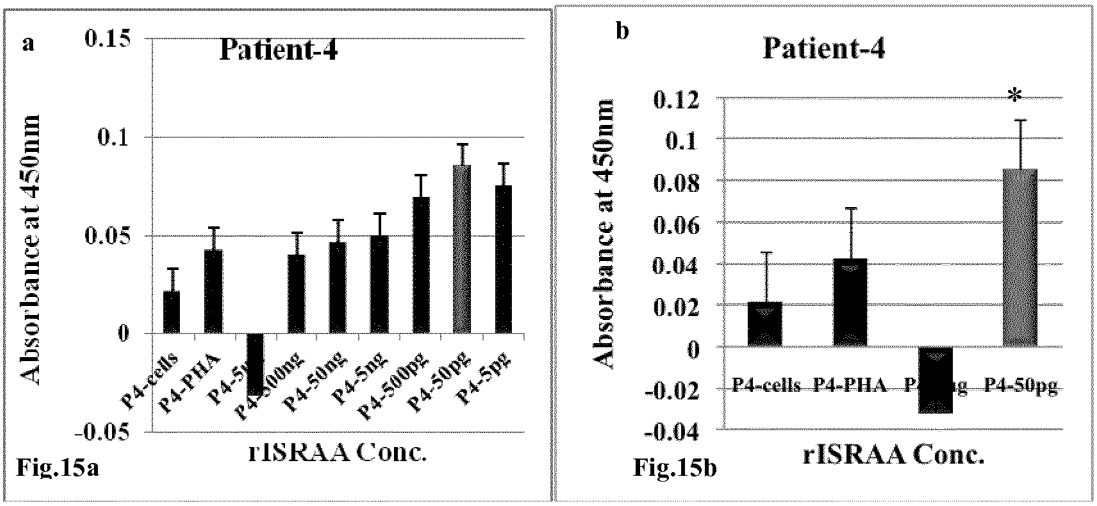


**Figure 16: Effect of rISRAA on Immunosuppressed Cells – Patient 5**

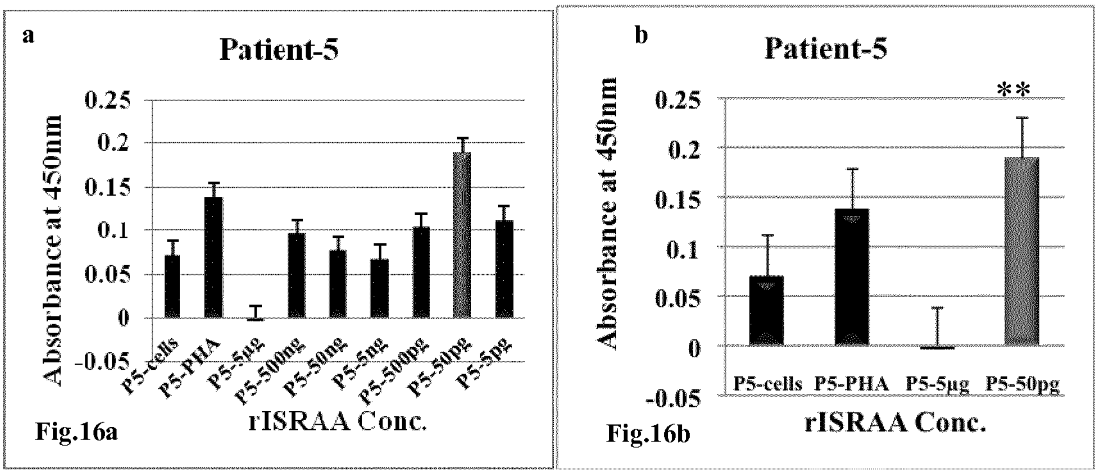


**Figure 17: Effect of rISRAA on Immunosuppressed Cells – Patient 6**

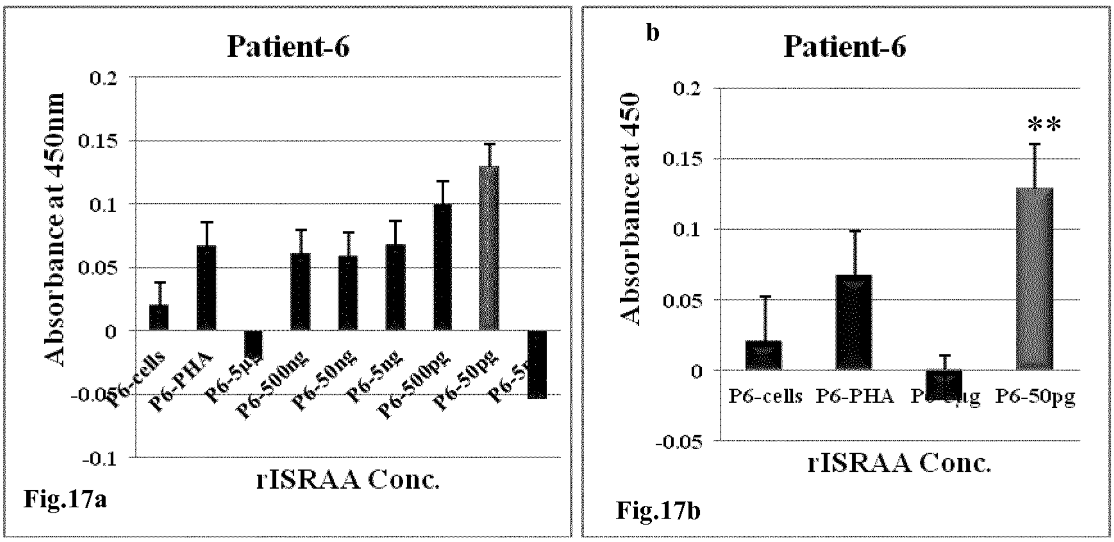

**Figure 18: Effect of rISRAA on Immunosuppressed Cells - Patient 7**

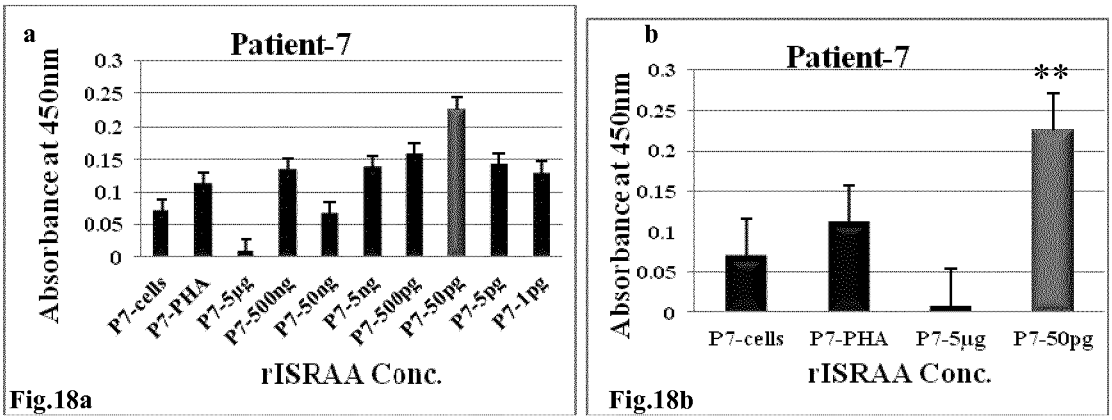


**Figure 19: Effect of rISRAA on Immunosuppressed Cells - Patient 8**

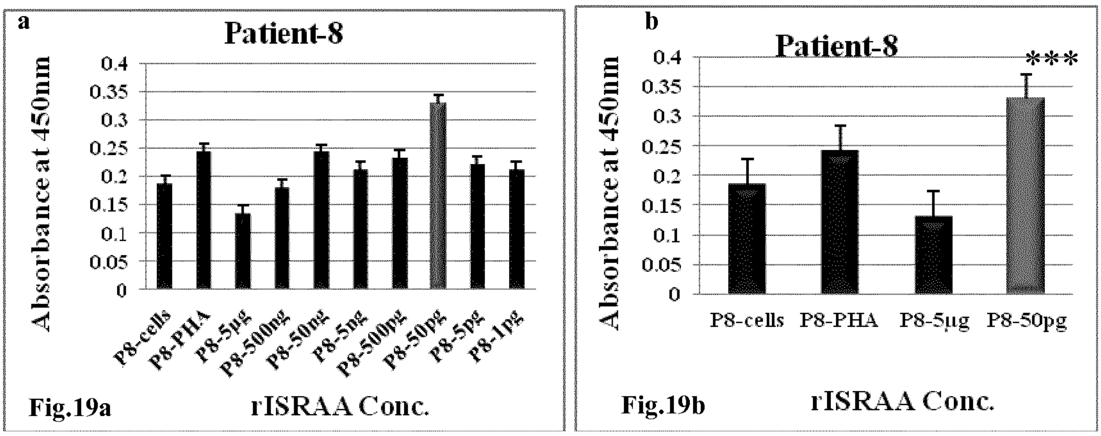

**Figure 20: In situ Cell Death Assay of immunosuppressed Cells**

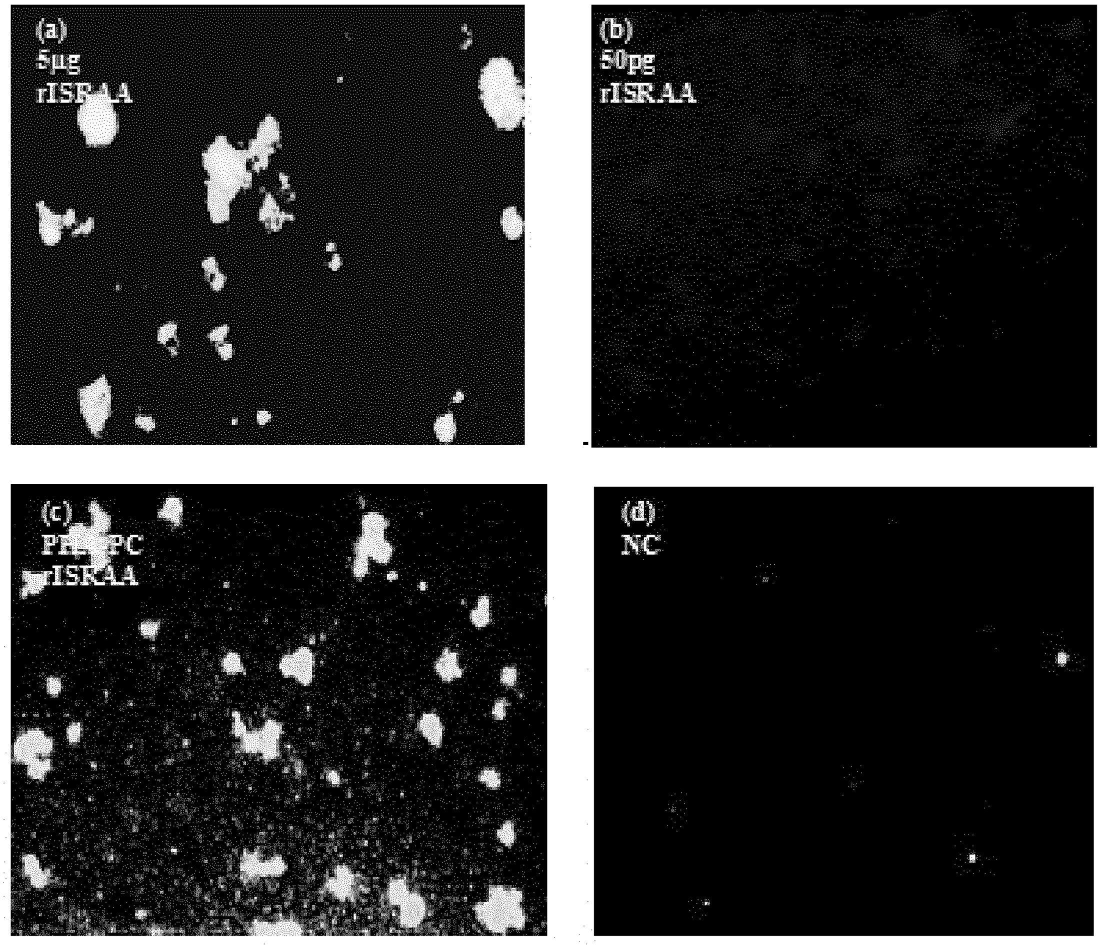

**Figure 21: Immunohistochemistry staining of proliferative marker Ki-67.**

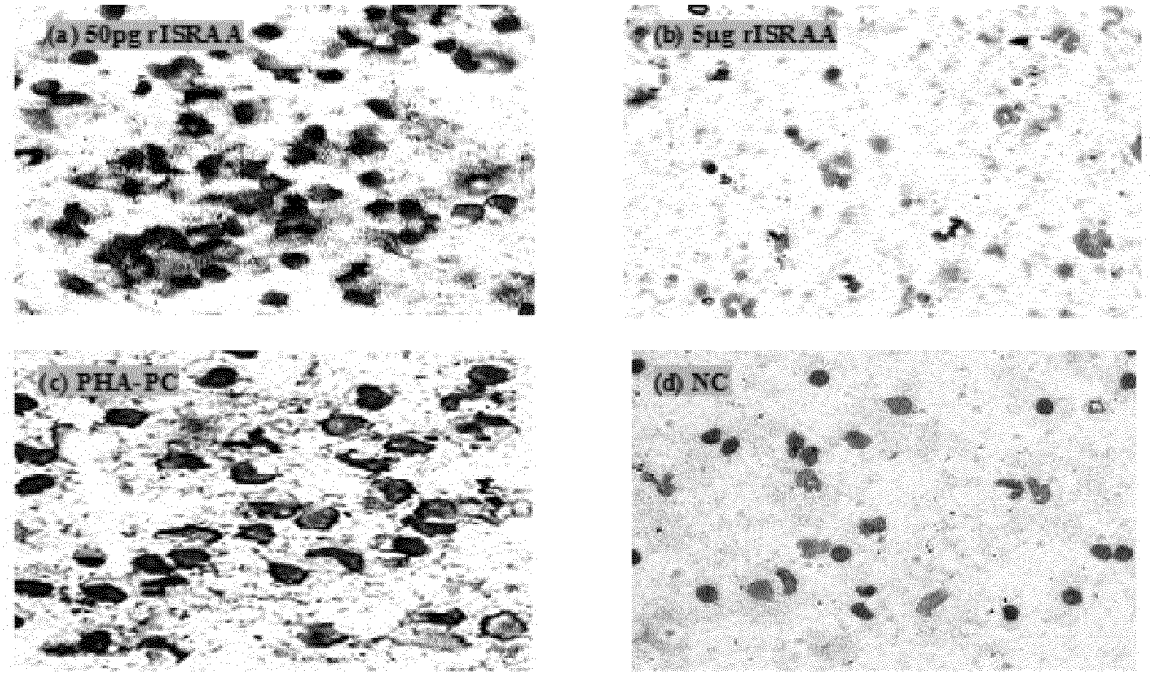

**Figure 22: ISRAA Treated U-973 cell line**

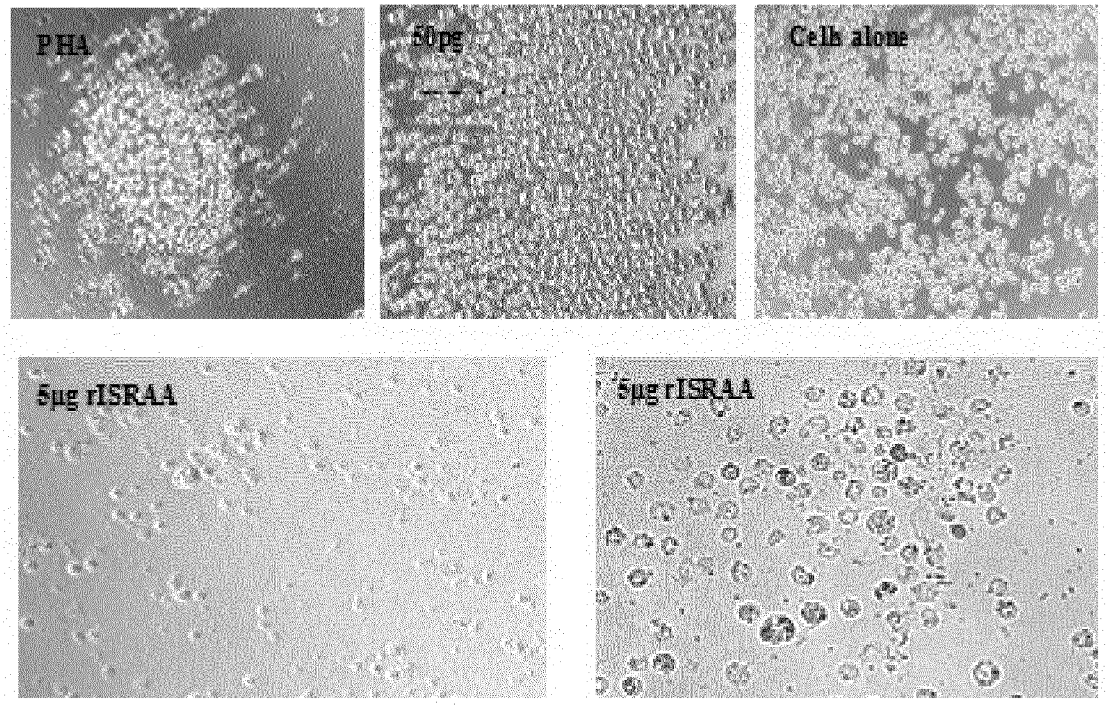

**Fiure 23: Cytocide rate of U-937 cell line (Histiocytic lymphoma)**

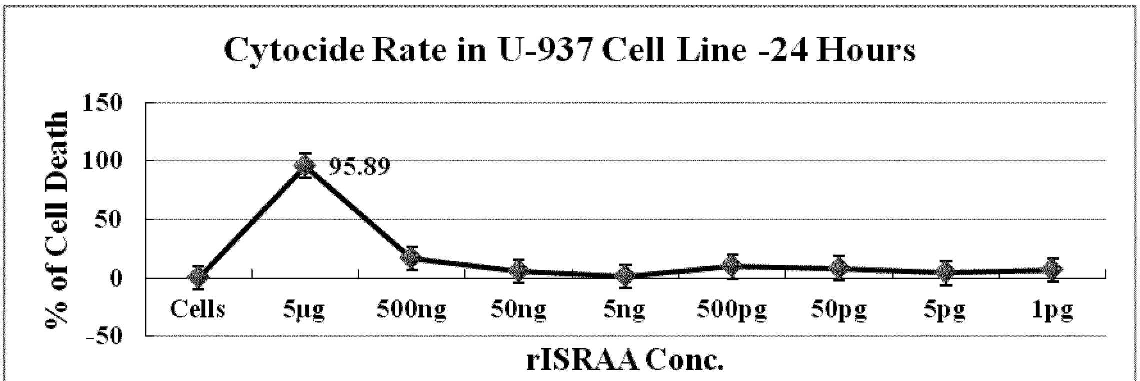


Fig.23 (a)

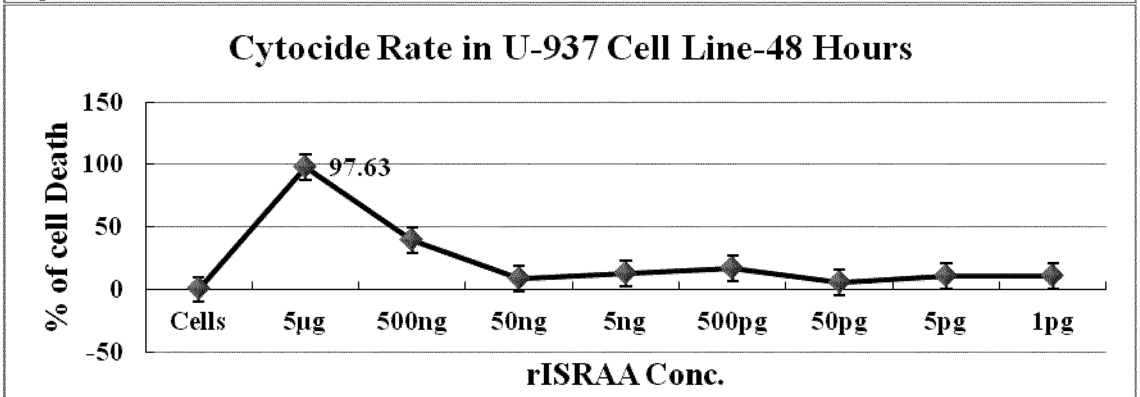


Fig.23 (b)

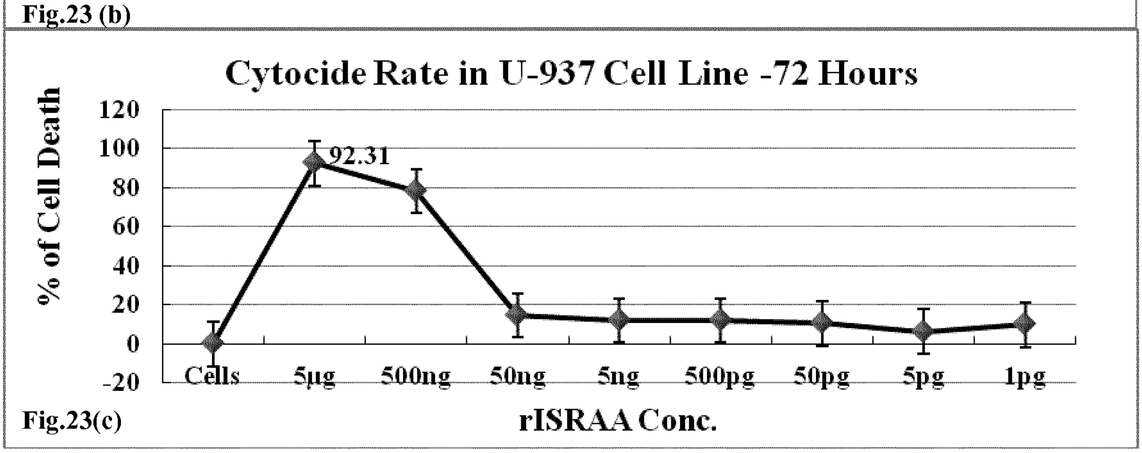


Fig.23(c)

Figure 24: In situ cell death assays of U-937 cell line.

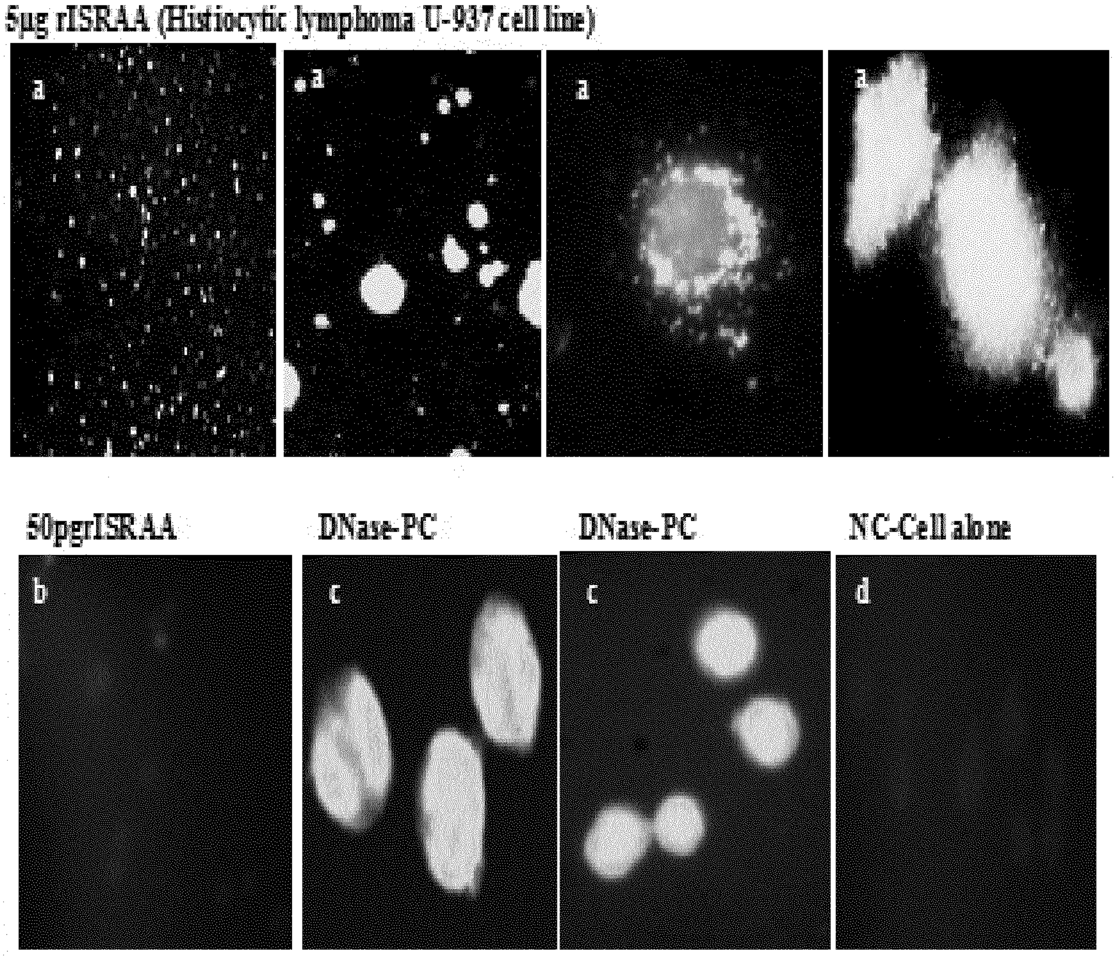

**Figure 25: Death Rate of Kelly cell line (Neuroblastoma).**

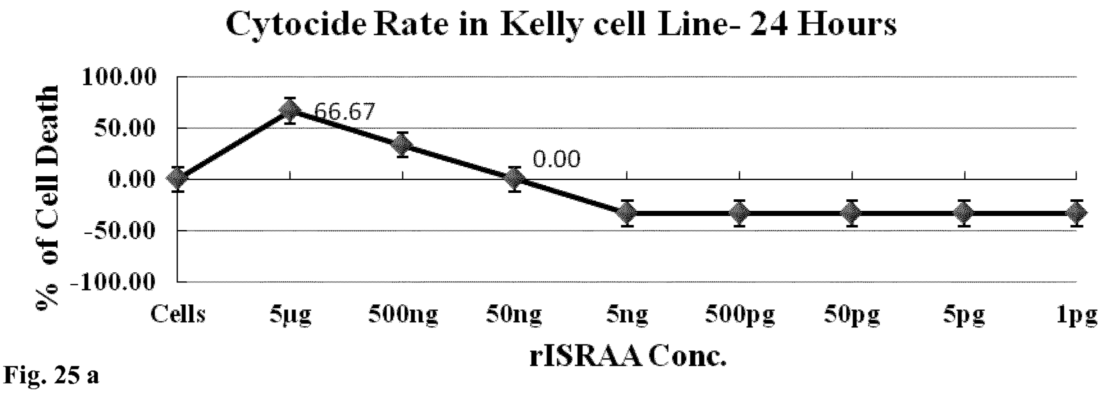


Fig. 25 a

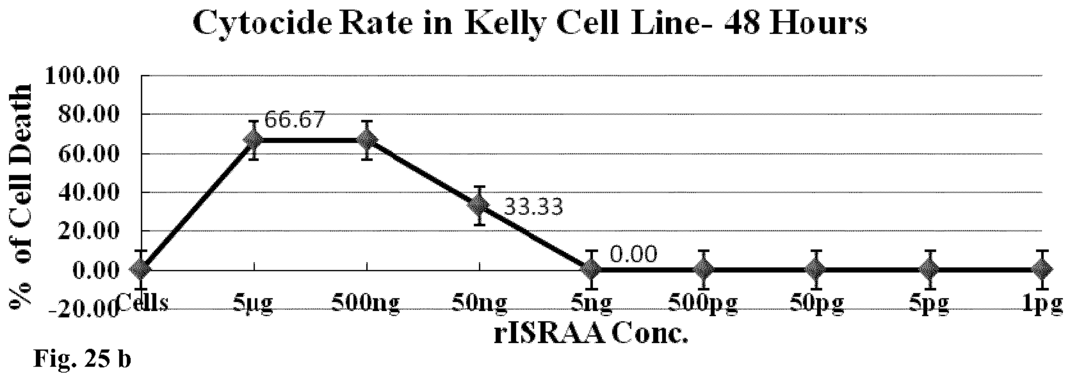


Fig. 25 b

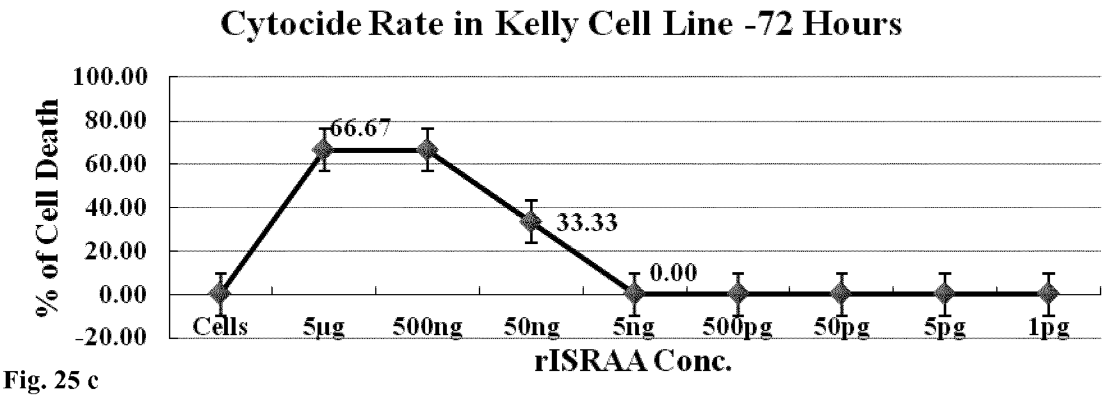


Fig. 25 c

**Figure 26: In situ cell death assays of Kelly cell line**

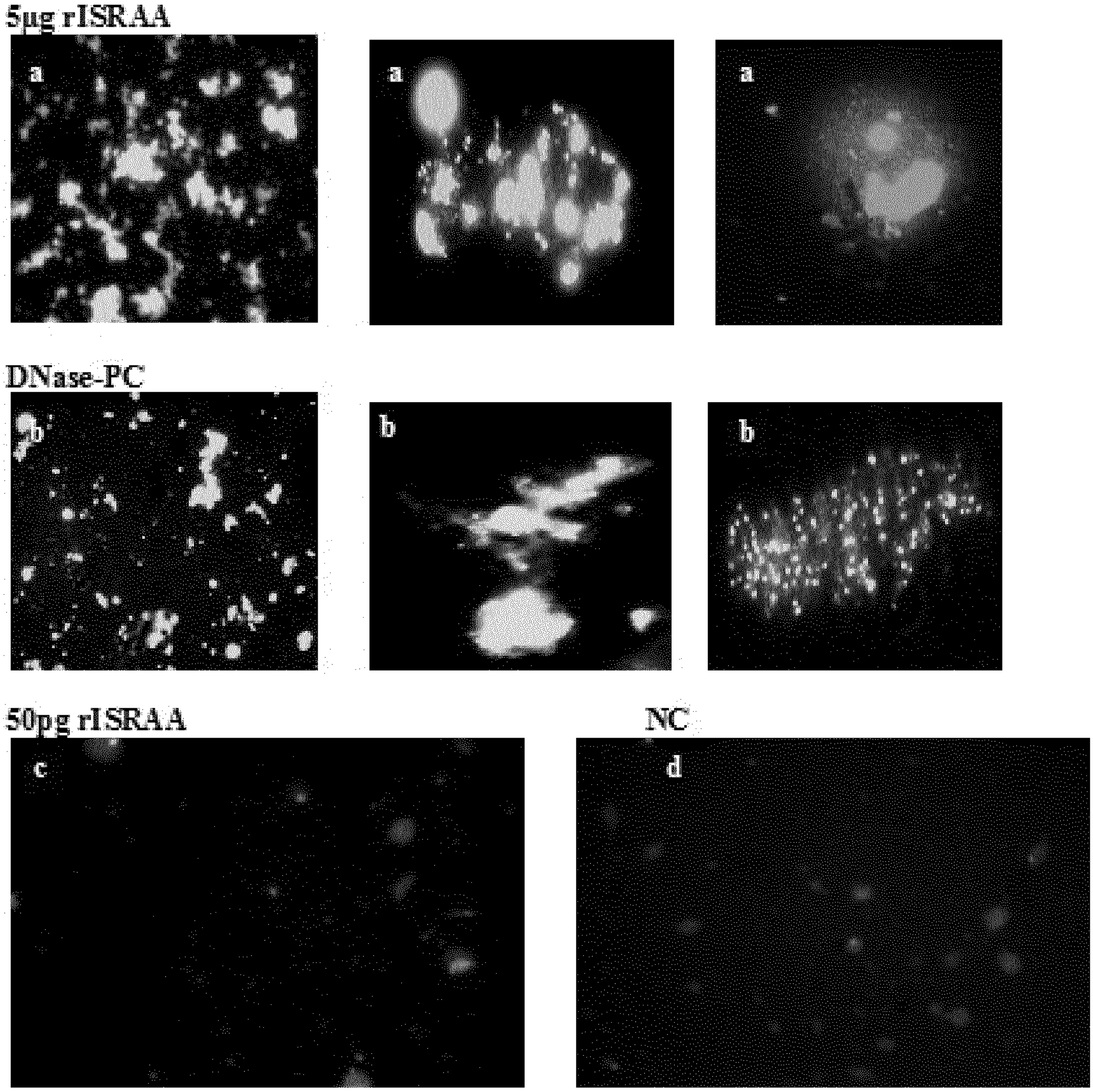

**Figure 27: Photographs of Papanicolaou Stain of rISRAA treated cells.**

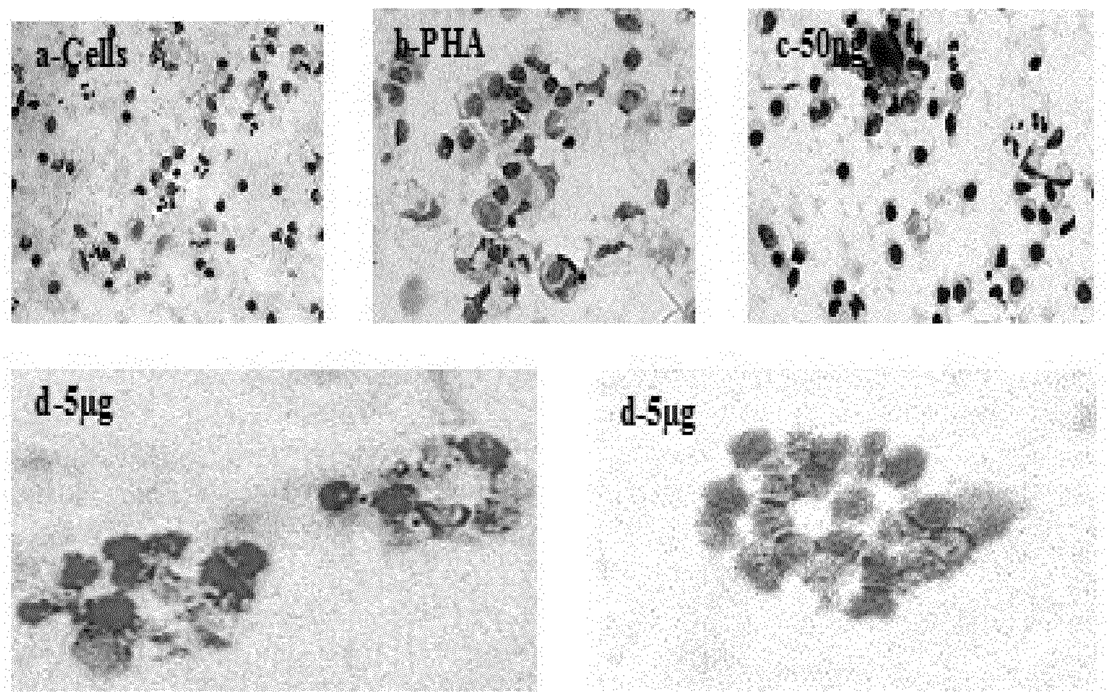

**Figure 28: Photographs of Giemsa stained cells.**

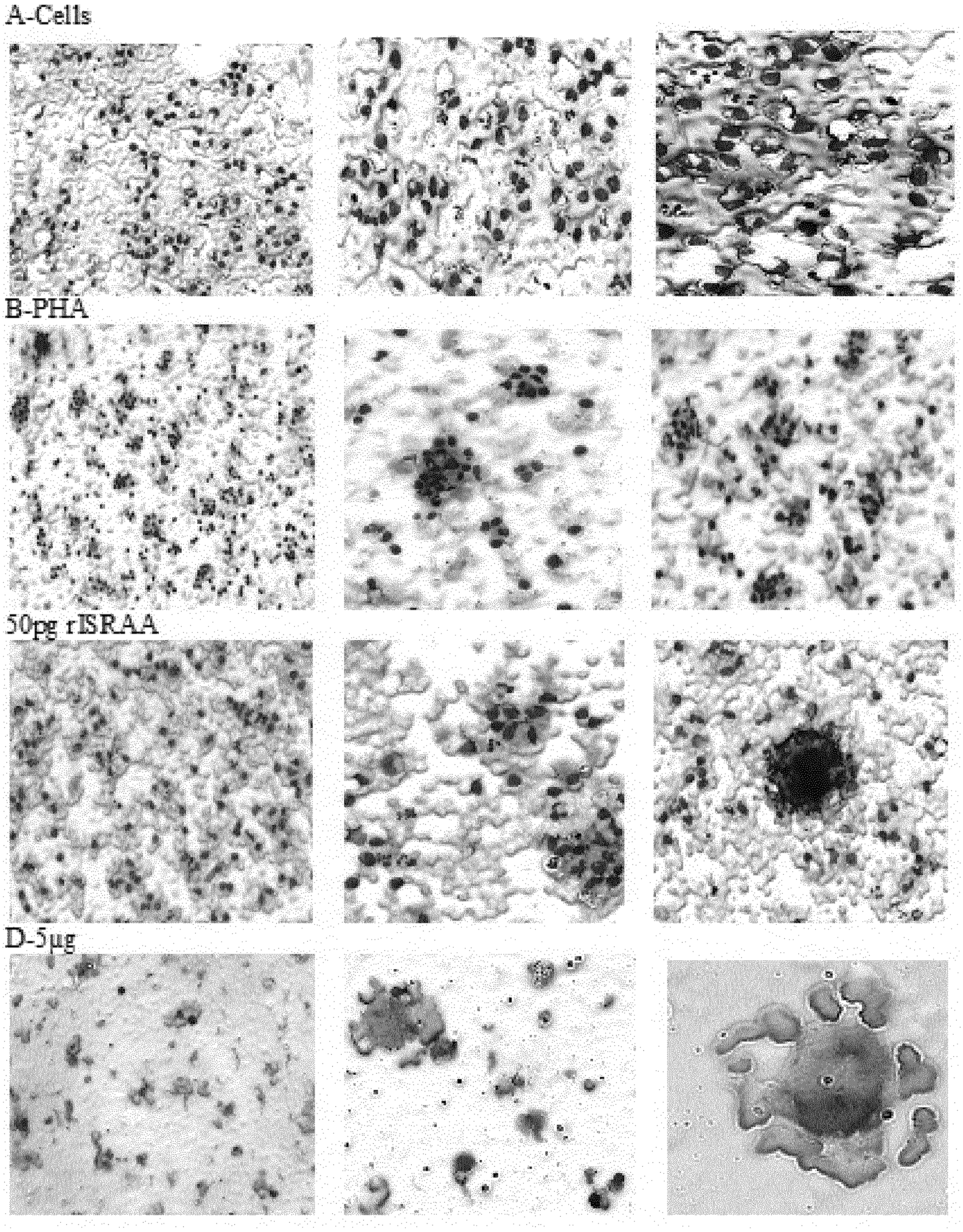

**Figure 29: Differentiation of peripheral blood stem cells treated with ISRAA**

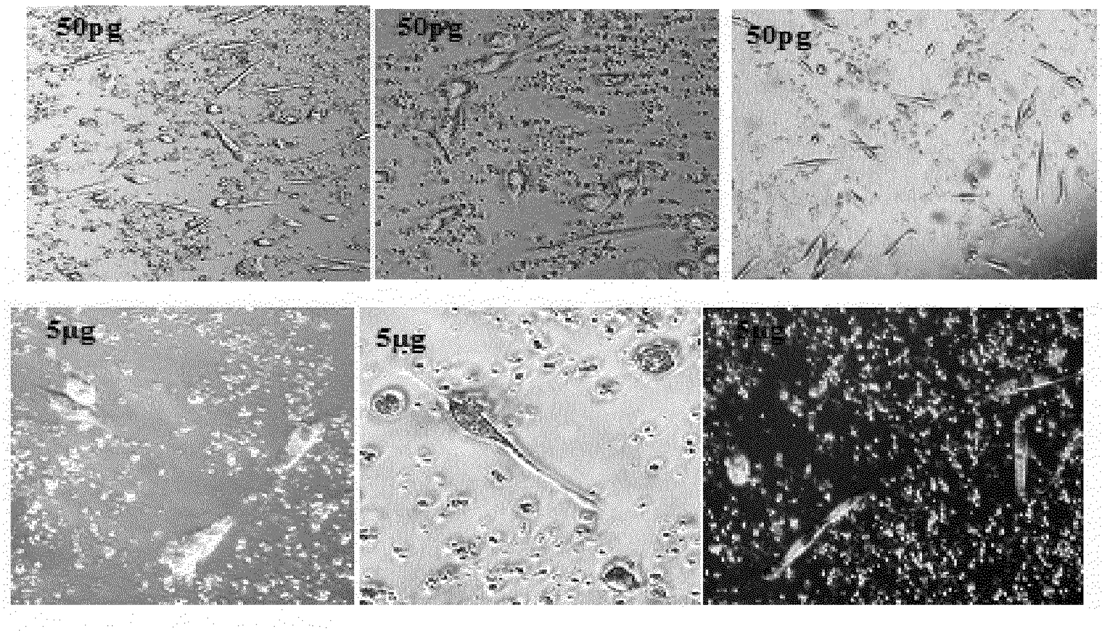

**Figure 30: Immunostaining of CD68 marker.**

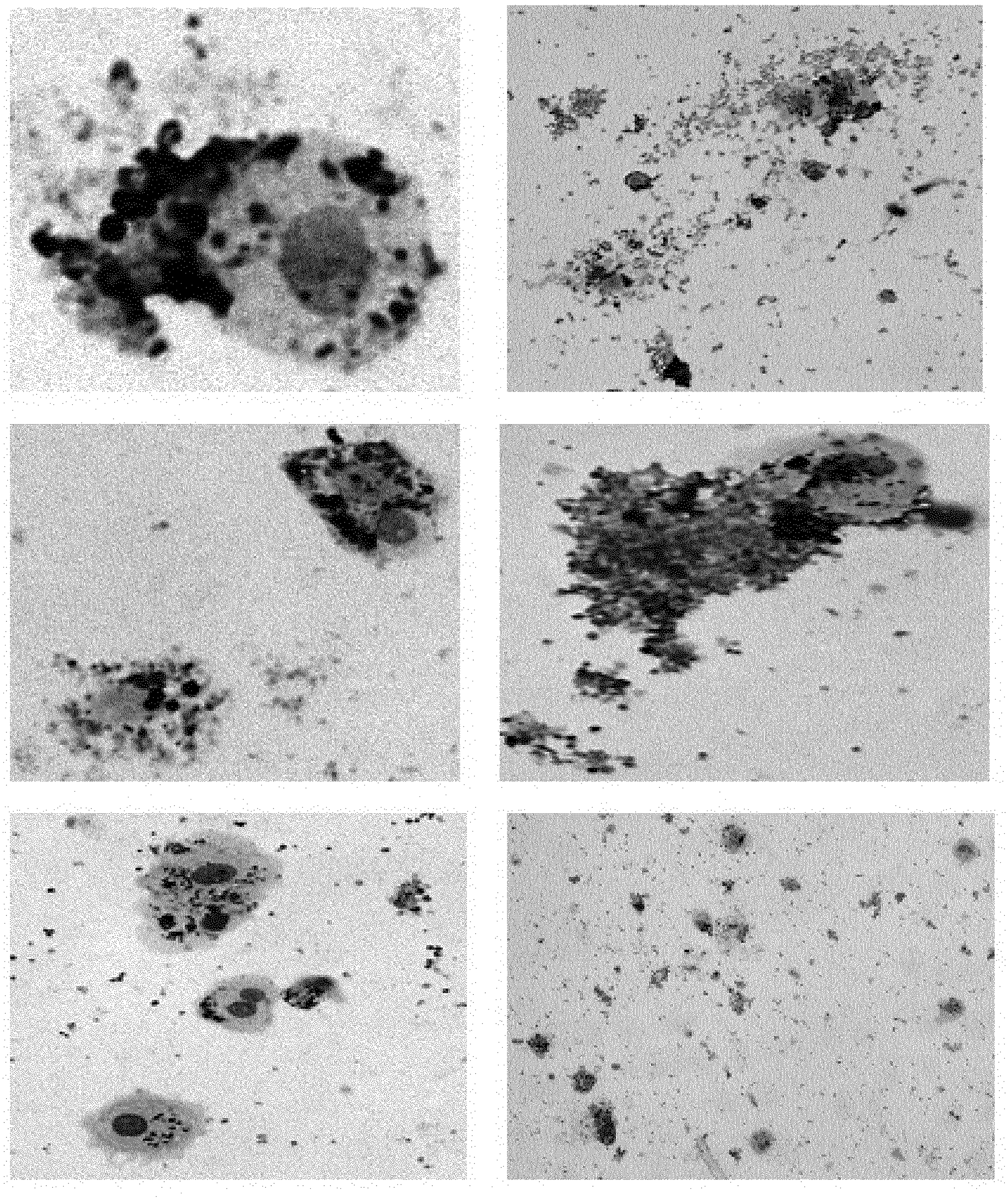

**Figure 31: Differentiation of embryonic brain cells, immunostaining of NF positive cells**

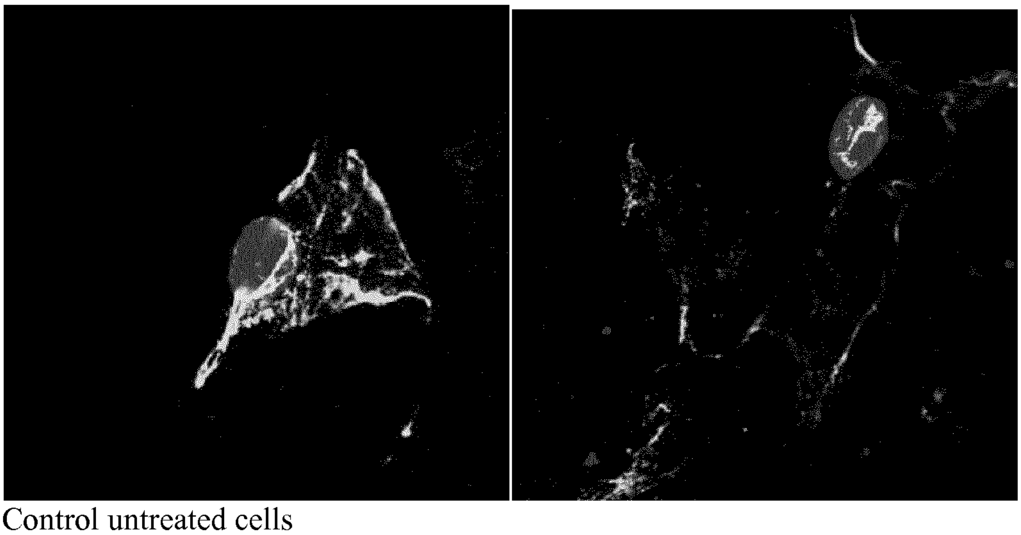

Control untreated cells

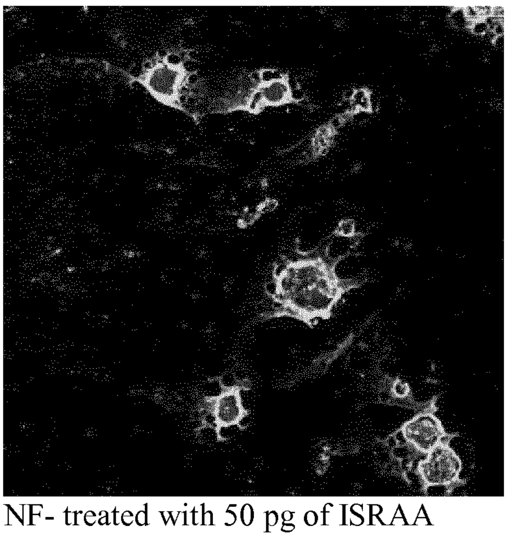

NF- treated with 50 pg of ISRAA

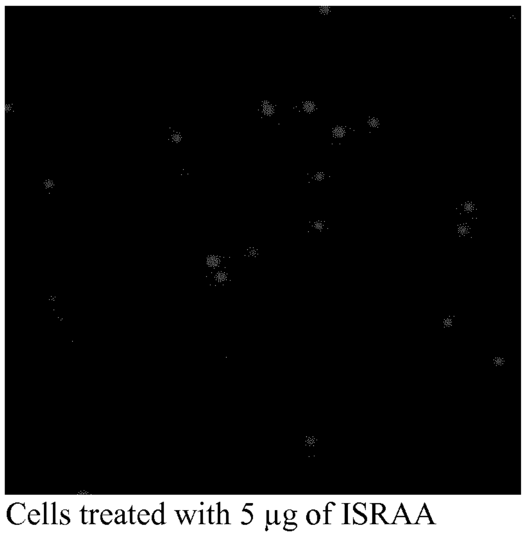

Cells treated with 5 µg of ISRAA

**Figure 32: Differentiation of embryonic brain cells, immunostaining of GFAP positive astrocytes cultured for 2 days.**

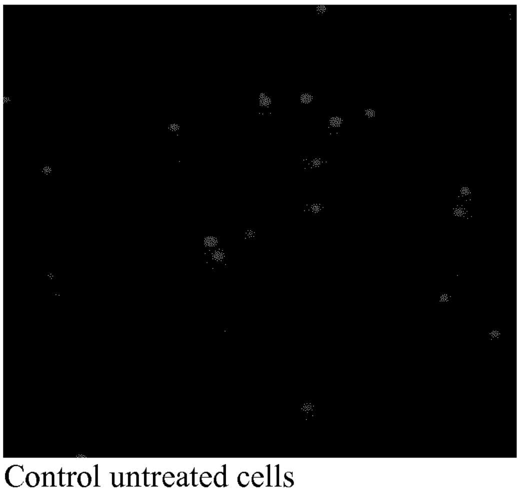

Control untreated cells

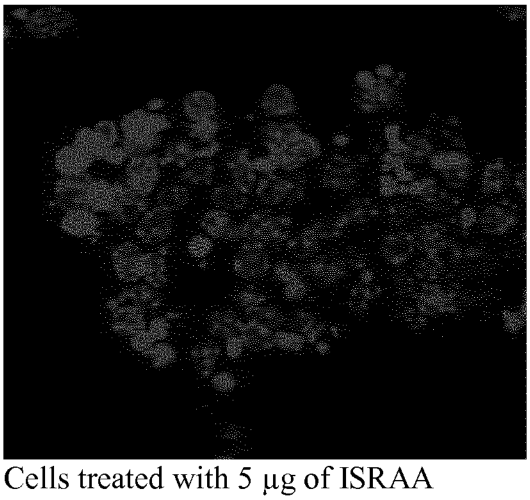

Cells treated with 5 µg of ISRAA

Cells treated with 50 pg of ISRAA

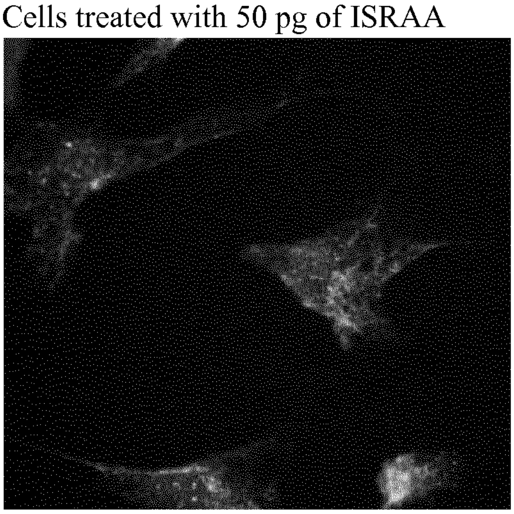

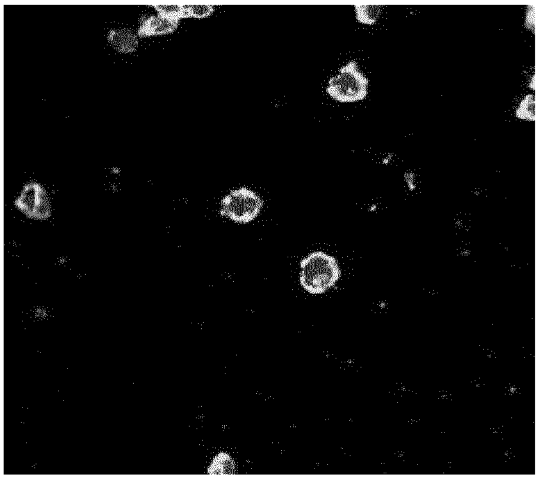

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200810475 A
- US 4816567 A
- US 5545807 A
- US 5545806 A
- US 5569825 A
- US 5625126 A
- US 5633425 A
- US 5661016 A
- GB 1121891 A


### Non-patent literature cited in the description


- **LIU, Y. et al.** *Tropical Medicine and International Health,* 1999, vol. 4 (2), 85-92
- **LIU, Y. et al.** *Scand. J. Immunol.,* 1999, vol. 50, 485-491
- **LIU, Y. et al.** *Neuroimmunomodulation,* 2004, vol. 11 (2), 113-118
- **BAKHIET, M. et al.** *Clin. Exp. Immunol.,* 2006, vol. 144 (2), 290-298
- **BAKHIET et al.** *Immunol Cell Biol,* 2008, vol. 86 (8), 688-99
- **ALTSCHUL et al.** *Methods in Enzymology,* 1996, vol. 266, 460-480
- **ALTSCHUL et al.** *Nucleic Acids Res.,* 1997, vol. 25, 3389-3402
- **CUNNINGHAM ; WELLS.** *Science,* 1989, vol. 244, 1081-5
- **BASS et al.** *Proc. Natl. Acad. Sci. USA,* 1991, vol. 88, 4498-502
- **HILTON et al.** *J. Biol. Chem.,* 1996, vol. 271, 4699-4708
- **DE VOS et al.** *Science,* 1992, vol. 255, 306-312
- **SMITH et al.** *J. Mol. Biol.,* 1992, vol. 224, 899-904
- **WLODAVER et al.** *FEBS Lett.,* 1992, vol. 309, 59-64
- **BARTON.** *Current Opin. Struct. Biol.,* 1995, vol. 5, 372-376
- **CORDES et al.** *Current Opin. Struct. Biol.,* 1996, vol. 6, 3-10
- **KOHLER ; MILSTEIN.** *Nature,* 1975, vol. 256, 495497
- **ZAPATA et al.** *Protein Eng.,* 1995, vol. 8 (10), 1057-1062
- **PLUCKTHUN.** The Pharmacology of Monoclonal Antibodies. Springer-Verlag, 1994, vol. 113, 269-315
- **JONES et al.** *Nature,* 1986, vol. 321, 522-525
- **RIECHMANN et al.** *Nature,* 1988, vol. 332, 323-329
- **PRESTA.** *Curr. Op. Struct. Biol.,* 1992, vol. 2, 593-596
- **RIECHMANN et al.** *Nature,* 1988, vol. 332, 323-327
- **VERHOEYEN et al.** *Science,* 1988, vol. 239, 1534-1536
- **HOOGENBOOM ; WINTER.** *J. Mol. Biol,* 1991, vol. 227, 381
- **MARKS et al.** *J. Mol. Biol,* 1991, vol. 222, 581
- **COLE et al.** Monoclonal Antibodies and Cancer Therapy. Alan R. Liss, 1985, 77
- **BOERNER et al.** *J. Immunol.,* 1991, vol. 147 (1), 86-95
- **MARKS et al.** *Bio/Technology,* 1992, vol. 10, 779-783
- **LONBERG et al.** *Nature,* 1994, vol. 368, 856-859
- **MORRISON.** *Nature,* 1994, vol. 368, 812-13
- **FISHWILD et al.** *Nature Biotechnology,* 1996, vol. 14, 845-51
- **NEUBERGER.** *Nature Biotechnology,* 1996, vol. 14, 826
- **LONBERG ; HUSZAR.** *Intern. Rev. Immunol.,* 1995, vol. 13, 65-93
- **DR. NESTOR VAN MEIRVENNE.** Laboratory of Serology. Institute of Tropical Medicine Prins Leopold

SEQUENCE LISTING

<110> Arabian Gulf University

<120> Applications of an immune system-released activating agent (ISRAA)

<130> 1154-0014

<140> GB 1121891.4
<141> 2011-12-20

<160> 3

<170> PatentIn version 3.3

<210> 1
<211> 2093
<212> DNA

<213> Homo sapiens

<400> 1

```
cccagttagt gcctgagtac gagagactca tcagggctga gaaaggagaa tggcagggga     60
aacagtgcta caaggatgtc ctttgtgtcc tgaccatgca gaaggagaca gaagccccag    120
gattggtggt tcccagaggc agagcccact cctggaggtg actcacactc agtgttgtca    180
tttgatcctt agctgtctgt atgtctggtg ccctgtgacc cagtctcttt atggtgctcc    240
ggagcagcat tcctttctgt ccccccttgt tcttctccac gcttcccaac gaccaggtac    300
acacccactt gccatctccc tgttaaccct accttcccag ttagtgcctg agtacacaga    360
agtgacatct ctccaggtca tcagaattct tgtcaatccc tctgagtcag caaactgcct    420
gggttagaag tctgggactt ctctgggact cagaacccaa tgcccagaat ctgagccaca    480
tcaaggctgg ttgttctccc tctcctcatc ttgagagttt ggcttgtgca tgacaggcct    540
gcctgcccac ttagttagcc ttccttcctt tcttcctaac ttcctgatgg ccttcgaagc    600
ccctagactt accatatcac taccaaaaga aaaaagaaaa agaaaaagct gtaagtggga    660
agtgacccac ttttgacccc tggtacatgc atcccaggta gatgccttgg tcccttgtct    720
tattggaact gattctagct catccatgag ctgaaggaag tcagtccttt gacccaagct    780
tgggactgtg gtgtcctggc tacattatct cccttctcac ccctcctcta gagggtcctt    840
caggggggcgg gctccccact gccagaggat aaagcatccc cgactccgga gccagccagc    900
catgccacag cactgtattt attggcggct cctcttcact caggcccacg acaggagttc    960
agacccccgc gagaaaagcc acccgctatt gatccttagt caggtctccg gagcctggca   1020
gacattcatc aaggagccac agcatggggc tacagctcgc cctttctttc ttcttccttt   1080
cttctacccc ccaagtcatt cccccccccc gggggggggt ccgagggagg cgggtgaggg   1140
ggcttgccga ggtcactcca agcactgcgg ggtggggtgg ggtggggtgg ggttggctgt   1200
gggccccggg cttttgagca agtctttgga agttagagat gtggaacttg gagcgagtca   1260
```

gggaaagccc ttctaacgaa gatagtgtgt ggagtgccgg catttctgta gactccaata 1320

ctaaaaagag gctgctaacc aagagggcct tgagggattt gattccagag aagagagcct 1380

agaaattagc aagtgaaagg ggccagtttg gactgatgaa tgggtgcttg aaaacaattt 1440

gttttcttaa gtatgtaaat atgctagtga agagtattta ctttttgggt taccttggaa 1500

aaccctgtat cttgaatatt atttccatcc tcagggtccc actcaggagc ttctgaagtg 1560

ggtcccgagg agaactaaga ggtaggagga cgaggaggca aaacaacggg aagctgtctt 1620

tataattttt aaaggattac caccaccaag tccctgcccc taaggtgtaa actttgctca 1680

gttccagcct gaatgttcac tcaacctgca ggaatgatct tttttaatat atgtatttca 1740

aaatatatgt atattttatt tatatatgta tatatcatgt atgaatatgt ttgcttgctt 1800

atatgtccgt gttccacttg tgtgtttggt gccctcggaa tagaagaggg tgttgaattt 1860

ccctaaacct ggagtttcag atgattgtga gctgccacat gggtgccaag gagagaattt 1920

tggccctttg caagagcaag aaaccttttt taaccactga gccatttttc ctgccccagg 1980

actttttttg taaagttagt tctttttgct actgtgtggg taagcccttg gccactgaac 2040

aggccctatg aatcagaaaa aaaaaaaaaa aaaaaaaaaa aaaaaaaaaa aaa 2093

<210> 2
<211> 378
<212> DNA
<213> Homo sapiens

<400> 2

atggcagggg aaacagtgct acaaggatgt cctttgtgtc ctgaccatgc agaaggagac 60

agaagcccca ggattggtgg ttcccagagg cagagcccac tcctggaggt gactcacact 120

cagtgttgtc atttgatcct tagctgtctg tatgtctggt gccctgtgac ccagtctctt 180

tatggtgctc cggagcagca ttcctttctg tccccccttg ttcttctcca cgcttcccaa 240

cgaccaggta cacacccact tgccatctcc ctgttaaccc taccttccca gttagtgcct 300

gagtacacag aagtgacatc tctccaggtc atcagaattc ttgtcaatcc ctctgagtca 360

gcaaactgcc tgggttag 378

<210> 3
<211> 125
<212> PRT
<213> Homo sapiens

<400> 3

```
Met Ala Gly Glu Thr Val Leu Gln Gly Cys Pro Leu Cys Pro Asp His
1               5                   10                  15

Ala Glu Gly Asp Arg Ser Pro Arg Ile Gly Gly Ser Gln Arg Gln Ser

            20                  25                  30

Pro Leu Leu Glu Val Thr His Thr Gln Cys Cys His Leu Ile Leu Ser
        35                  40                  45

Cys Leu Tyr Val Trp Cys Pro Val Thr Gln Ser Leu Tyr Gly Ala Pro
    50                  55                  60

Glu Gln His Ser Phe Leu Ser Pro Leu Val Leu Leu His Ala Ser Gln
65                  70                  75                  80

Arg Pro Gly Thr His Pro Leu Ala Ile Ser Leu Leu Thr Leu Pro Ser
                85                  90                  95

Gln Leu Val Pro Glu Tyr Thr Glu Val Thr Ser Leu Gln Val Ile Arg
            100                 105                 110

Ile Leu Val Asn Pro Ser Glu Ser Ala Asn Cys Leu Gly
        115                 120                 125
```